# EUROPEAN PATENT APPLICATION

(11) **EP 1 582 223 A1**
(43) Date of publication of application: **05.10.2005**
(21) Application number: 03768321.6
(22) Date of filing: 26.12.2003
(51) Int. Cl.: A61K 48/00, A61K 38/02, A61K 31/7088, A61P 43/00, G01N 33/53, C12N 15/16

(54) **VECTOR FOR GENE THERAPY AND METHOD OF QUANTIFYING TARGET PROTEIN IN MAMMAL OR CULTURED CELLS WITH THE ADMINISTRATION OF THE VECTOR FOR GENE THERAPHY**

(30) Priority: 10.01.2003 JP 2003003967
(71) Applicant: Niigata TLO Corporation, Niigata 950-2181 (JP)
(72) Inventor: HANAWA, Haruo, Nishiohata Shokuinshukusha RA205, Niigata-shi, Niigata 951-8104 (JP)
(74) Representative: Tollervey, Rebecca Marie
(86) International application number: PCT/JP2003/016956
(87) International publication number: WO 2004/062693

(57) **Abstract**

Disclosed is a vector for gene therapy, by which the blood level of a desired protein can be monitored at high sensitivity when a gene therapy is conducted, wherein the label peptide does not have a physiological activity and has no immunogenicity in many mammals. The vector for gene therapy comprises a nucleic acid coding for a fusion protein of glucagon C-terminal side 19-29 amino acid peptide region and a desired protein region which should be produced in the body, which nucleic acid is incorporated into an expression vector for mammalian cells.

## Description

### Technical Field

The present invention relates to a vector for gene therapy for the production of a desired protein in the body, which vector enables to simply quantify the desired protein produced in the body or in cultured cells.

### Background Art

When a gene therapy by which a desired protein is made to be produced in the body of a patient, the blood level of the desired protein cannot be measured unless a method for measuring the protein, such as ELISA, has been established. To counter this problem, an assay method in which a label protein is used and the level of the protein is measured has been practically used and sold. However, the measurement sensitivity of the method is low, and so there is no established method for measuring blood level in gene therapy. In Treatment of Murine Lupus with cDNA encoding IFN-γ R/Fc, The Journal of Clinical Investigation, July 2000, volume 106, Number 2 p207-215, the desired protein is not measured but a protein influenced by the desired protein is quantified so as to indirectly prove the expression of the desired protein. This is thought to show the difficulty in the measurement of the blood level.

### Disclosure of the Invention

An object of the present invention is to provide a vector for gene therapy, by which the blood level of a desired protein can be monitored at high sensitivity when a gene therapy is conducted, and by which undesired physiological action or antigen-antibody reaction due to the label scarcely occurs.

The present inventor intensively studied to discover that by carrying out the gene therapy with a vector in which a fusion protein of the desired protein which should be produced in the body by the gene therapy and a glucagon C-terminal side 19-29 amino acid peptide, the blood level of the desired protein may be measured with high sensitivity using the glucagon peptide as a label, and that undesired physiological action or induction of immunological reaction due to the label peptide scarcely occurs, thereby completing the present invention.

That is, the present invention provides a vector for gene therapy comprising an expression vector for mammalian cells and a nucleic acid coding for a fusion protein of glucagon C-terminal side 19-29 amino acid peptide region and a desired protein region which should be produced in the body, which vector can produce the fusion protein in the mammalian cells. The present invention also provides a method for gene therapy comprising administering an effective amount of the vector for gene therapy according to the present invention to a mammal or cultured mammalian cells, in which expression of the desired protein in the body or in the cultured mammalian cells is desired. The present invention further provides a use of the vector for gene therapy according to the present invention for the production of a drug for gene therapy. The present invention still further provides a method for quantifying a desired protein produced in the body or in cultured cells by expression of the vector for gene therapy, comprising quantifying, by immunoassay, the glucagon C-terminal side 19-29 amino acid peptide region in a test sample collected from a mammal or cultured mammalian cells to which the vector for gene therapy according to the present invention was administered. The present invention still further provides a label for labeling a desired protein produced by expression of an externally administered expression vector in the body of a mammal or in cultured mammalian cells, consisting essentially of glucagon C-terminal side 19-29 amino acid peptide. The present invention still further provides a method for labeling a protein produced in the body or in cultured cells, comprising labeling a desired protein produced in the body or in cultured cells with glucagon C-terminal side 19-29 amino acid peptide by expressing the desired protein produced by expression of an externally administered expression vector in the body of a mammal or in cultured mammalian cells, as a fusion protein with the glucagon C-terminal side 19-29 amino acid peptide as a label. The present invention still further provides a use of glucagon C-terminal side 19-29 amino acid peptide as a label for a desired protein produced by expression of an externally administered expression vector in the body of a mammal or in cultured mammalian cells.

By the present invention, a vector for gene therapy by which the blood level of the desired protein can be measured with high sensitivity, wherein the label peptide does not have a physiological action, was first provided. Since the glucagon C-terminal side 19-29 itself does not have a physiological action and is well conserved in various mammals, it does not substantially induce an immunological reaction while it can be quantified by immunoassay with high sensitivity using a commercially available immunoassay kit.

### Brief Description of the Drawings

Fig. 1 shows the nucleotide sequence of the nucleic acid fragment inserted into the vector for gene therapy prepared in Example 1, together with the amino acid sequence encoded thereby.
Fig. 2 shows the continuation of Fig. 1.
Fig. 3 shows the continuation of Fig. 2.
Fig. 4 shows a gene map of pCAGGS which is an expression vector for mammals used in Examples 1 to 5.
Fig. 5 shows the relationship between the days after administration of the vector for gene therapy and the blood level of the desired protein measured by measuring the glucagon-originated label peptide, in Example 1.
Fig. 6 shows the change with time of the blood level of the desired protein when gene therapy was carried out using the vector of the present invention, and shows the change with time of the blood glucose level when gene therapy was carried out using the vector of the present invention or using a vector in which a nucleic acid coding for the desired protein not fused with the glucagon-originated label peptide was inserted.
Fig. 7 shows the nucleotide sequence of the nucleic acid fragment inserted into the vector for gene therapy prepared in Example 2, together with the amino acid sequence encoded thereby.
Fig. 8 shows the continuation of Fig. 7.
Fig. 9 shows the continuation of Fig. 8.
Fig. 10 shows the relationship between the days after administration of the vector for gene therapy and the blood level of the desired protein measured by measuring the glucagon-originated label peptide, in Example 2.
Fig. 11 shows the days for which the transplanted hearts were taken by the rats in Example 2 and Comparative Example 2.
Fig. 12 shows the nucleotide sequence of the nucleic acid fragment inserted into the vector for gene therapy prepared in Example 3, together with the amino acid sequence encoded thereby.
Fig. 13 shows the continuation of Fig. 12.
Fig. 14 shows the continuation of Fig. 13.
Fig. 15 shows the nucleotide sequence of the nucleic acid fragment inserted into the vector for gene therapy prepared in Comparative Example 3, together with the amino acid sequence encoded thereby.
Fig. 16 shows the continuation of Fig. 15.
Fig. 17 shows the relationship between the days after administration of the vector for gene therapy and the blood level of the desired protein measured by measuring the glucagon-originated label peptide, in Example 3.
Fig. 18 shows the percentage of the lesion area of myocarditis in rats in Example 3 and Comparative Example 3.
Fig. 19 shows the nucleotide sequence of the nucleic acid fragment inserted into the vector for gene therapy prepared in Example 4, together with the amino acid sequence encoded thereby.
Fig. 20 shows the continuation of Fig. 19.
Fig. 21 shows the continuation of Fig. 20.
Fig. 22 shows the relationship between the days after administration of the vector for gene therapy and the blood level of the desired protein measured by measuring the glucagon-originated label peptide, in Example 4.
Fig. 23 shows the percentage of the lesion area of rat myocarditis in Example 4 and Comparative Example 4.
Fig. 24 shows the nucleotide sequence of the nucleic acid fragment inserted into the vector for gene therapy prepared in Example 5, together with the amino acid sequence encoded thereby.
Fig. 25 shows the relationship between the blood level of the desired protein measured by measuring the glucagon-originated label peptide and the blood level of the desired protein measured by measuring human interleukin 8, in Example 5.

### Best Mode for Carrying Out the Invention

The "glucagon C-terminal side 19-29 amino acid peptide" which is expressed by the vector according to the present invention in the form of a fusion protein, means the peptide consisting essentially of totally 11 amino acids located from the 19th to 29th amino acid counted from the C-terminal of glucagon. That is, the peptide has the amino acid sequence shown in SEQ ID NO: 1 in SEQUENCE LISTING. Since the "glucagon C-terminal side 19-29 amino acid peptide" is used as a label of the desired protein, the peptide may be hereinafter referred to as "glucagon-originated label peptide" for convenience.

In the vector according to the present invention, a nucleic acid coding for a fusion protein of the glucagon-originated label peptide region and a desired protein region is incorporated.

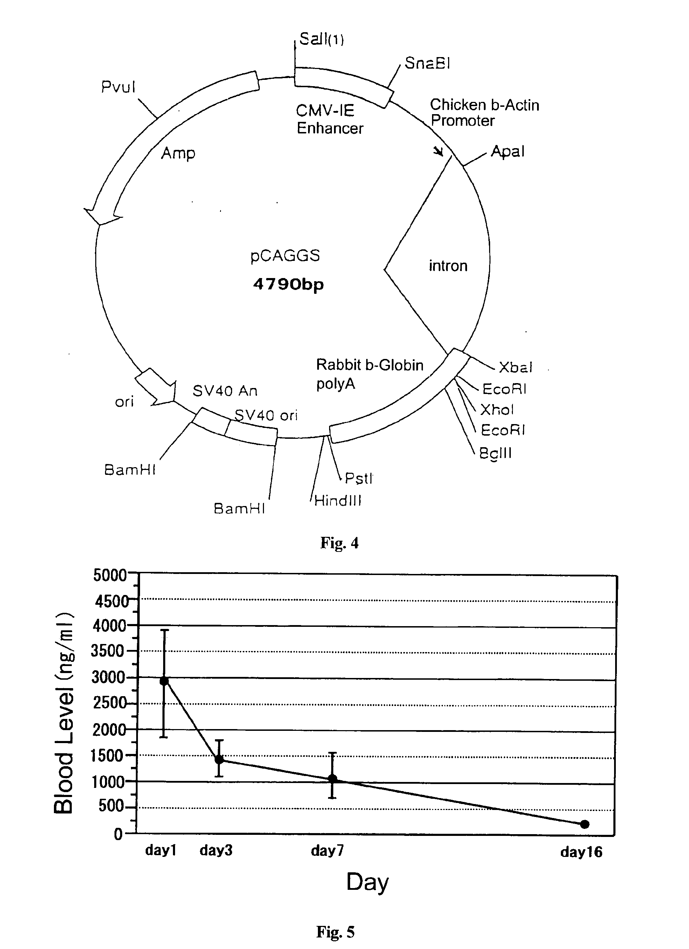

The expression vector for mammalian cells used in the present invention is well-known in the field of gene therapy, and is not restricted as long as it is an expression vector for mammalian cells. The characteristic feature of the present invention resides in that the glucagon-originated label peptide region as a label of the desired protein is expressed in the form of a fusion protein with the desired protein, so that the expression vector for mammalian cells is not restricted at all, and any of the known expression vectors for mammalian cells used in the field of gene therapy may be employed. Although the vector may be a plasmid vector or a virus vector, a plasmid vector is preferred from the view point of safety. Various expression vectors for mammalian cells are well-known and are commercially available, and the well-known or commercially available vectors may preferably be employed. Examples of the well-known or commercially available vectors include pCAGGS (Efficient selection for high expression transfactans with a novel eukaryotic vector, Gene 1991 Dec.15,108(2)p193-P199., whose gene map is shown in Fig. 4, and whose nucleotide sequence is shown in SEQ ID NO: 3 in SEQUENCE LISTING), pCI vectors, pSI vectors and pTARGET vectors from PROMEGA, and pcDNA5/TO and the like from INVITROGEN, but the vectors are not restricted thereto.

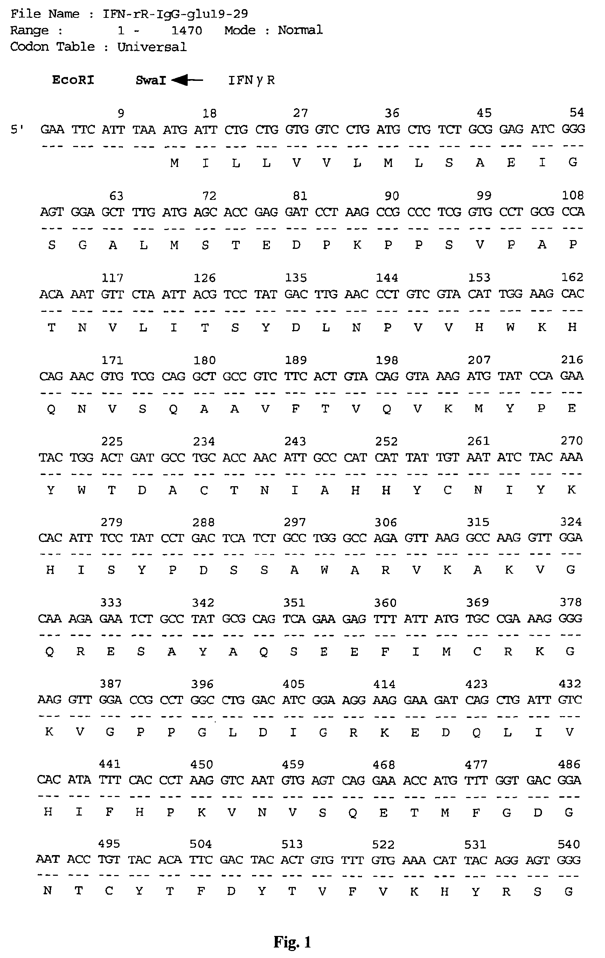

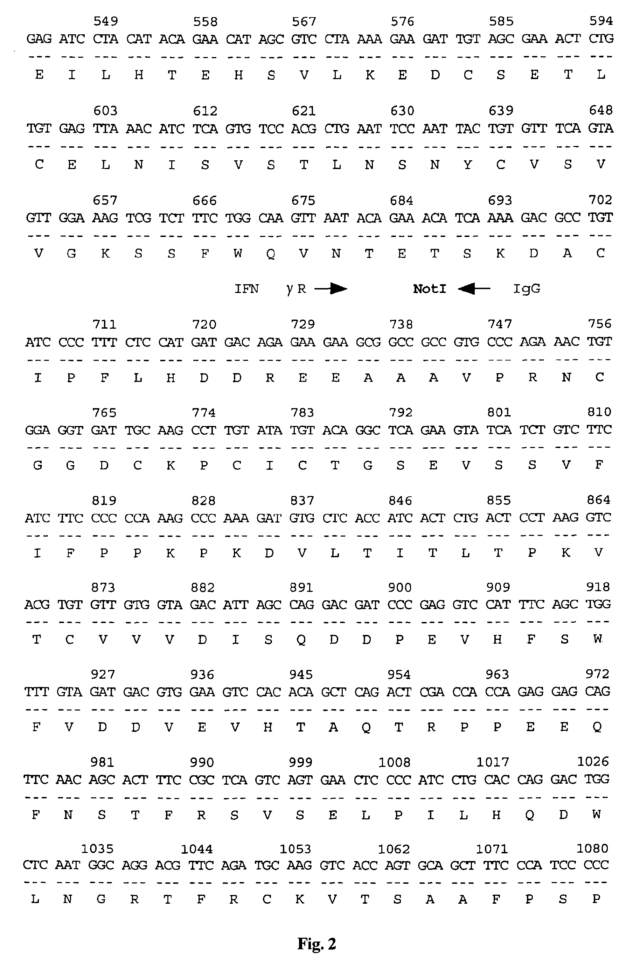

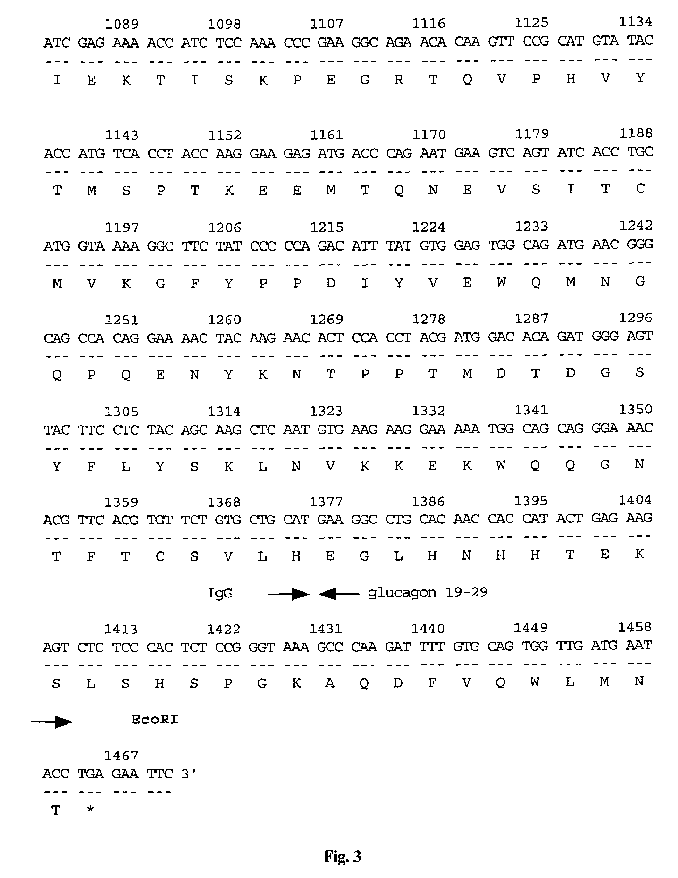

The desired protein to be produced in the body by the gene therapy is not restricted at all, and examples thereof include various cytokines such as interferons, interleukins and CTLA4; hormones such as growth factor and insulin; and cell adhesion factors; as well as receptors thereof. An arbitrary antigenic protein may also be produced in the body by a gene vaccine. The desired protein itself may also be a fusion protein. For example, a terminal of a desired cytokine which is desired to be bound to a Fc receptor may be fused with an immunoglobulin, preferably IgG, especially the constant region (Fc) of IgG1 so as to promote the binding ability with the Fc receptor, and the resulting fusion protein may be employed as the desired protein (see Examples 1 to 4 below). The nucleotide sequence coding for the Fc region of IgG is well-known. For example, the nucleotide sequence of the nucleic acid coding for the Fc region of human IgG is described in, for example, GenBank Accession No. BC020823 and so on, and the nucleotide sequence of the nucleic acid coding for the Fc region of rat IgG is shown in Figs. 1 to 3 of the present application, and so on.

The vector according to the present invention may be obtained by inserting the nucleic acid coding for the fusion protein of the desired protein and the above-described label peptide into a cloning site of the expression vector for mammalian cells. It is preferred to fuse the label peptide to a terminal, especially to the C-terminal of the desired protein.

Gene therapy may be carried out by administering the vector for gene therapy according to the present invention to a mammal. As the administration route, a parenteral route such as intravenous injection or intramuscular injection is preferred. The dose of the vector may be appropriately selected depending on the properties of the desired protein, the type and the severity of the disease to be treated, and the dose of the vector per 1 kg of body weight may be usually about 1 mg to 10 mg, preferably about 2 mg to 4 mg. As for the formulation, the vector for gene therapy may be dissolved in Ringer solution and the obtained solution may be used as an injection solution. Additives for injection solution, well-known in the field of drug formulation may also be added. Alternatively, the vector for gene therapy according to the present invention may be administered to mammalian cells cultured *in vitro.* More particularly, there is a gene therapy in which the cells such as lymphocytes and bone marrow cells from a patient are collected from the body and cultured, a gene vector is administered to the cultured cells, and the cells acquired the ability to produce the desired protein are returned again to the patient. The vector for gene therapy according to the present invention may also be administered to such cultured mammalian cells. Alternatively, the vector for gene therapy may be administered to cultured mammalian cells in experiments for examining the therapeutic effect, *in vitro,* of the vector for gene therapy or the like.

In the gene therapy, the fusion protein of the above-described desired protein and the glucagon-originated label peptide is produced by the vector introduced into the body. Alternatively, in cases where the vector for gene therapy is administered to mammalian cells cultured *in vitro,* the fusion protein of the above-described desired protein and the glucagon-originated label peptide is produced in the cultured cells. Since the desired protein is fused with the label peptide, the level of the desired protein can be measured by measuring the level of the glucagon-originated label peptide. Since kits (including an antibody obtained by using the glucagon-originated label peptide as an antigen) for quantifying, by immunoassay, the glucagon-originated label peptide used in the present invention are commercially available (Pancreas Glucagon RIA kit commercially available from DAIICHI RADIOISOTOPE LABORATORIES, LTD and the like), the glucagon-originated label peptide may easily be measured using such a commercially available immunoassay kit.

The test sample for which the glucagon-originated label peptide is quantified may be various body fluids or tissues or dilutions thereof, collected from the individual to which the vector for gene therapy according to the present invention was administered, and preferably blood samples such as whole blood, serum or plasma as well as dilutions thereof. Alternatively, in cases where the vector for gene therapy is administered to the mammalian cells cultured *in vitro,* the test sample may be the homogenate of the cultured cell, culture supernatant or the like.

### Example 1, Comparative Example 1

PCR was carried out using as a template the cDNA of cultured rat spleen cells stimulated by lection, and using as primers 5'-gagaattcatttaaatgagagcggccgccgtgcccagaaactgtg-3' and 5'-tcaaccactgcacaaaatcttgggctttacccggagagtgggagagact-3'. Thereafter, PCR was performed using as the template the 300-fold diluted PCR product obtained above, and using as primers 5'-gagaattcatttaaatgagagcggccgccgtgcccagaaactgtg-3' and 5'-gagagagagaattctcaggtattcatcaaccactgcacaaaatcttgggc-3'. The amplified product was inserted into a cloning site of the above-described expression vector pCAGGS for mammalian cells. By this, pCAGGS-IgG-glu19-29 (pCAGGS in which a nucleic acid fragment having a region coding for the Fc region of immunoglobulin G1 (IgG1) and a region coding for the glucagon-originated label peptide, ligated to the downstream of the Fc-coding region, was inserted at the EcoRI site of pCAGGS), having restriction sites of SwaI and NotI, was obtained.

Thereafter, PCR was performed using as a template the cDNA of heart of rat suffering from myocarditis, and using as primers 5'-gagaattcatttaaatgattctgctggtggtcctgatg-3' and 5'-gcagcatcgcggccgcttcttctctgtcatcatggagaaa-3'. The obtained PCR product was inserted into the pCAGGS-IgG-glu 19-29 prepared above using SwaI and NotI.

By this, a vector according to the present invention, in which the DNA fragment having a nucleotide sequence shown in SEQ ID NO:2 in SEQUENCE LISTING (shown together with the restriction sites) was inserted into the EcoRI site of the above-described expression vector pCAGGS for mammalian cells was prepared (Example 1). SEQ ID NO:2, together with other information, is shown in Figs. 1 to 3. As shown in Figs. 1 to 3, the inserted nucleic acid fragment (IFNγR-IgG-glucagon¹⁹⁻²⁹) had EcoRI sites at both ends, in which a region coding for the glucagon-originated label peptide was ligated to the downstream of the region coding for the fusion protein of interferon γ receptor (IFNγR) protein and Fc region of immunoglobulin G1 (IgG1).

A plasmid vector not containing the glucagon-originated label peptide (Comparative Example 1) and the plasmid vector (Example 1) according to the present invention prepared as described above, which contained the glucagon-originated label peptide, were intravenously administered by jet injection to 7 rats, respectively, from the tail vein, thereby performing gene therapy. The injection solution was one obtained by dissolving 800 µg per rat of the plasmid dissolved in 20 ml of Ringer solution. After the injection, blood was sampled with time, the sampled 1 to 10 µl of plasma was 100-fold to 1000-fold diluted, and the level of the glucagon-originated label peptide, and in turn, the level of the desired protein (in this Example, the IFNγR/IgG1Fc fusion protein) was measured using a commercially available RIA kit (Pancreas Glucagon RIA kit commercially available from DAIICHI RADIOISOTOPE LABORATORIES, LTD and the like) in accordance with the instructions attached to the kit. That is, more concretely, RIA was performed as follows: To 400 µl of buffer for assay, 200 µl of standard glucagon solution or diluted test plasma was added, and 100 µl of glucagon-¹²⁵I solution and 100 µl of glucagon anti-serum solution were added, followed by leaving the resultant to stand at 4°C for 48 hours. Thereafter, 100 µl of a second antibody and 400 µl of a precipitation stabilizer for glucagon RIA were added, and the resulting mixture was left to stand at 4°C for 30 minutes. The resultant was then centrifuged (2000 x g, 30 minutes, 4°C), and the count rate was measured to determine the level after removing the supernatant.

Fig. 5 shows the results of the measurement of the blood level. In Example 1, on Day 1 after intravenous injection, the level was 2870 ± 1062 ng/ml (mean ± standard deviation), on Day 3, the level was 1440 ± 334ng/ml, on Day 7, the level was 1120 ± 433 ng/ml, and on Day 16, the level was 281 ± 162 ng/ml, and the level was able to be measured in all cases. On the other hand, in the gene therapy with the plasmid vector not containing the glucagon peptide (Comparative Example 1), the level was less than the detection limit in all cases by the same assay.

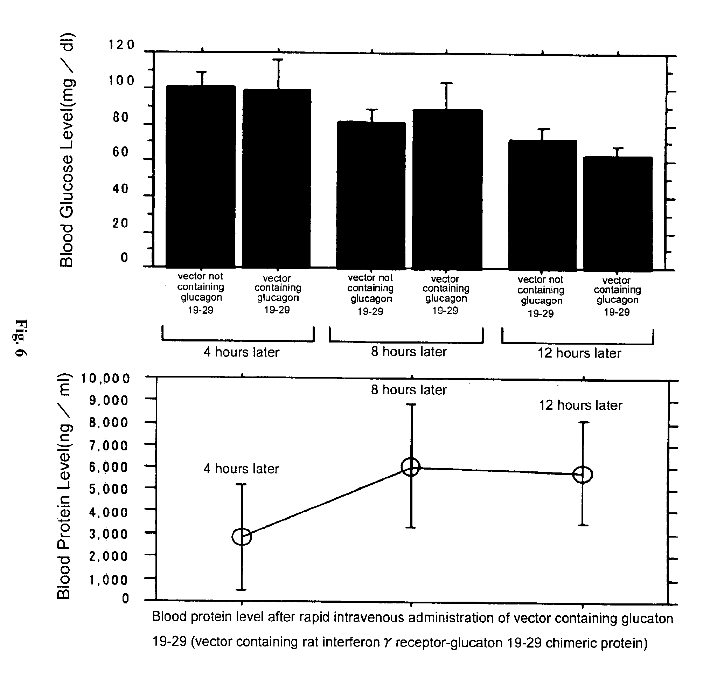

Fig. 6 shows blood glucose level and the blood protein level measured by the RIA as described above, at 4, 8 and 12 hours after the intravenous injection of the plasmid. In Example 1, the blood level was 2815 ± 2318 ng/ml at 4 hours later, 6061 ± 2789 ng/ml at 8 hours later, and 5752 ± 2270 ng/ml at 12 hours later. The blood glucose levels at 8 to 12 hours later in which the blood protein level was the maximum, were 89.3 ± 15.1 mg/dl (Example 1) vs. 81.8 ± 7.5 mg/dl (Comparative Example 1), and 63.5 ± 5.7 mg/dl (Example 1) vs. 71.4 ± 6.9 mg/dl (Comparative Example 1), so that there were no differences.

From the results described above, it was shown that the blood level of the desired protein can be well measured until several tens days after the administration of the vector, using a very small quantity of plasma sample.

### Example 2, Comparative Example 2

PCR was carried out using as a template the cDNA of cultured rat spleen cells stimulated by lectin, and using as primers 5'-gagaattcatttaaatggcttgtcttggactccagagg-3' and 5'-gcagcatcgcggccgcgtctgaatctgggcatggttctgg -3'. The obtained PCR product was incorporated into pCAGGS-IgG-glu19-29 prepared by the method described in Example 1, using SwaI and NotI.

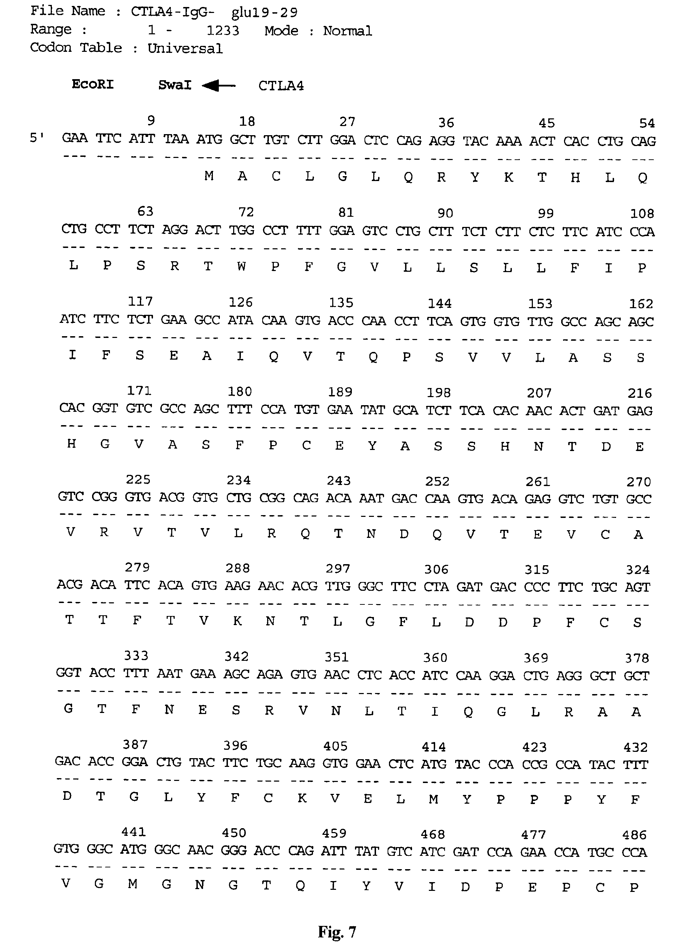

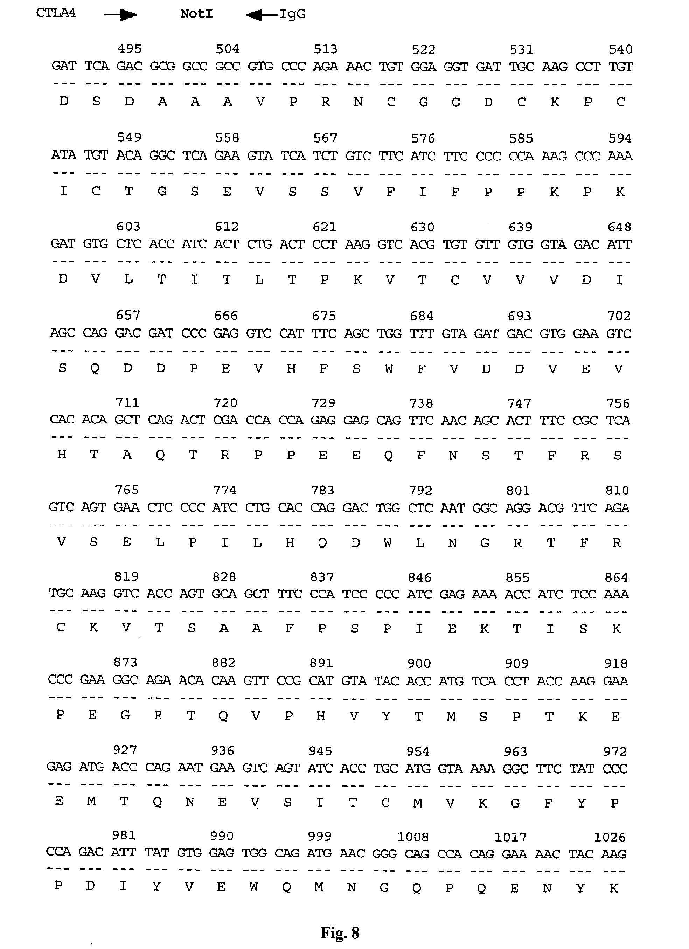

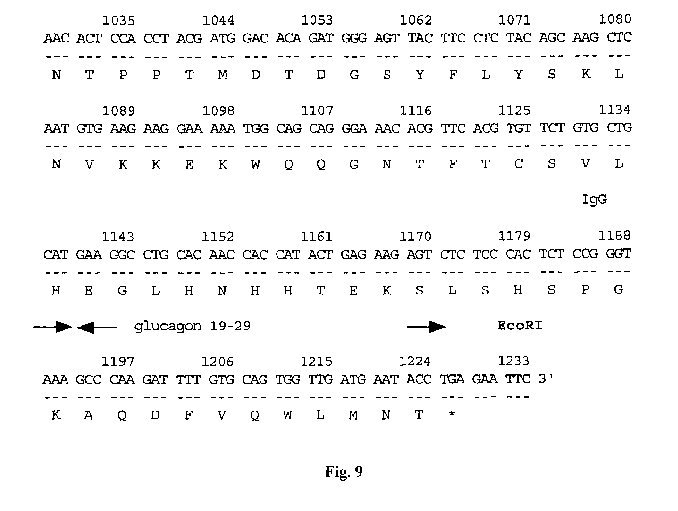

By this, a recombinant vector containing rat CTLA4-IgG-glucagon¹⁹⁻²⁹ (a nucleic acid fragment in which the rat IgG Fc-coding region was ligated to the downstream of rat CTLA4-coding region, and the glucagon-originated label peptide-coding region was ligated to the downstream of the rat IgG Fc-coding region) having the nucleotide sequence shown in Figs. 7 to 9 and in SEQ ID NO:4 (the restriction sites are also shown), inserted into the EcoRI site of the above-described expression vector pCAGGS for mammalian cells, which recombinant vector expresses CTLA4-IgG-glucagon¹⁹⁻²⁹ in rat cells was prepared (Example 2). For comparison, a recombinant vector in which IgG-glucagon¹⁹⁻²⁹-coding region alone not containing the CTLA4-coding region was inserted was also prepared (Comparative Example 2).

To each rat after heart transplantation, the recombinant vector was administered in the same manner as in Example 1, and the blood level was measured. The days during which the heart was taken were also checked.

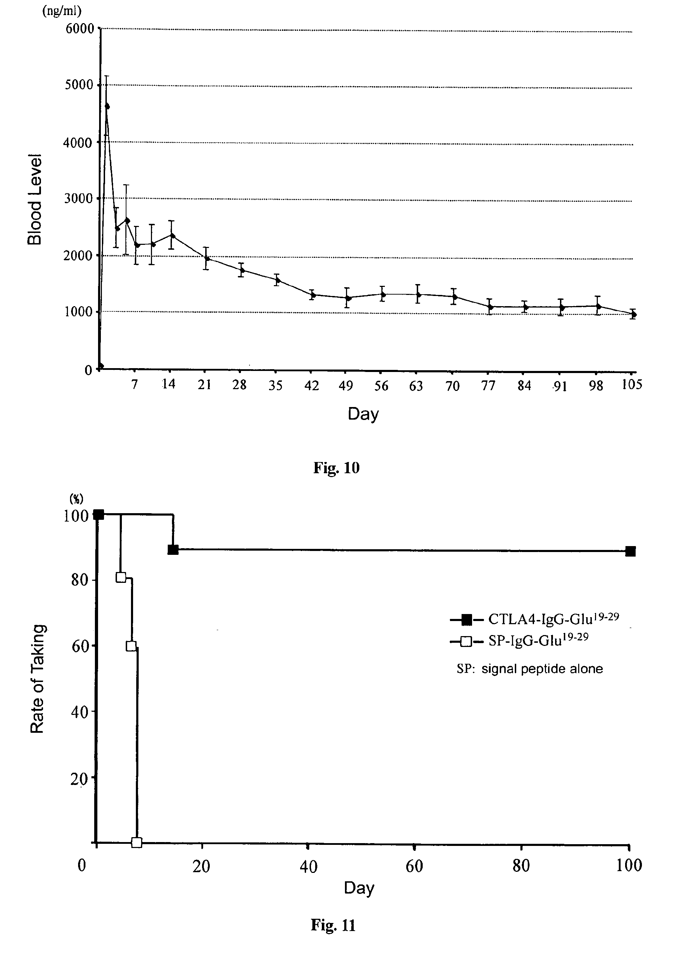

The results are shown in Figs. 10 and 11. As shown in Fig. 10, in Example 2, the CTLA4-IgG-glucagont¹⁹⁻²⁹ protein level changed as shown in Fig. 7. That is, although the glucagon in a 100-fold diluted sample could not be measured before the administration, the level sharply increased on Day 1 to a protein level of exceeding 5000 ng/ml. Although the protein level gradually decreased thereafter, the protein level was kept at more than 1000 ng/ml until the rats were sacrificed for evaluation on Day 105. As shown in Fig. 11, in Example 2, the heart was rejected in one animal among 10 animals on Day 14, in all of the remaining 9 animals, the heart was taken until Day 105 on which evaluation was performed. In the group treated with pCAGGS-SP-IgG-glucagon¹⁹⁻²⁹ not containing CTLA4, the heart of one animal among 5 animals was rejected on Day 5, the heart of one animal was rejected on Day 6, and the hearts of 3 animals were rejected on Day 7. These results show significant effectiveness of the therapy by pCAGGS-CTLA4-IgG-glucagon¹⁹⁻²⁹.

### Example 3, Comparative Example 3

PCR was carried out using as a template the cDNA of cultured rat spleen cells stimulated by lectin, and using as primers 5'-gagaattcatttaaatggcactctgggtgactgcagtc-3' and 5'-gcagcatcgcggccgcgtggccatagcggaaaagttgctt-3'. The obtained PCR product was incorporated into pCAGGS-IgG-glu19-29 prepared by the method described in Example 1, using SwaI and NotI.

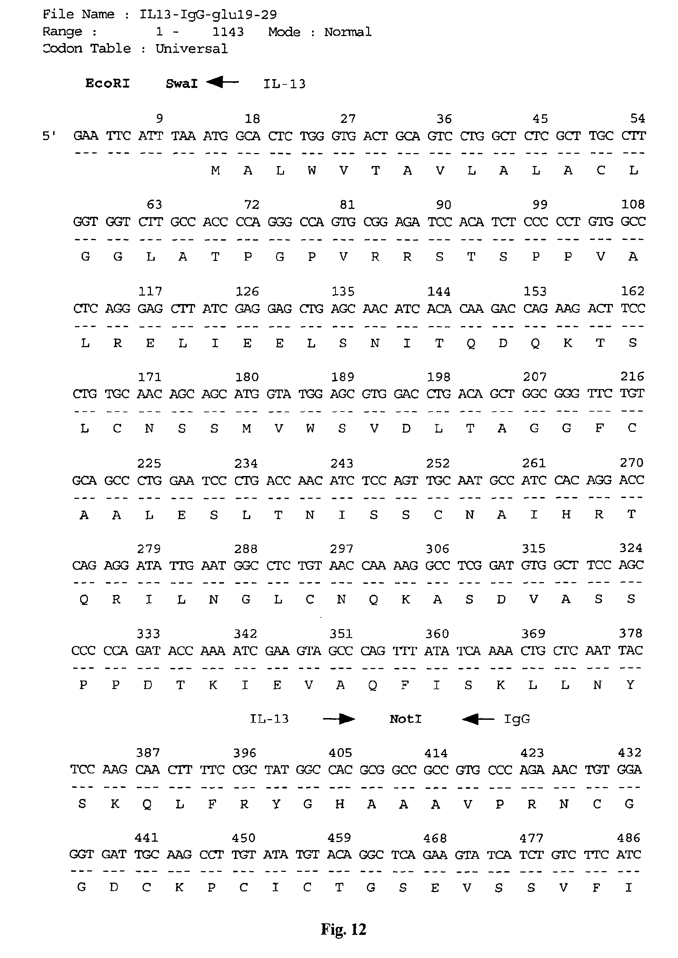

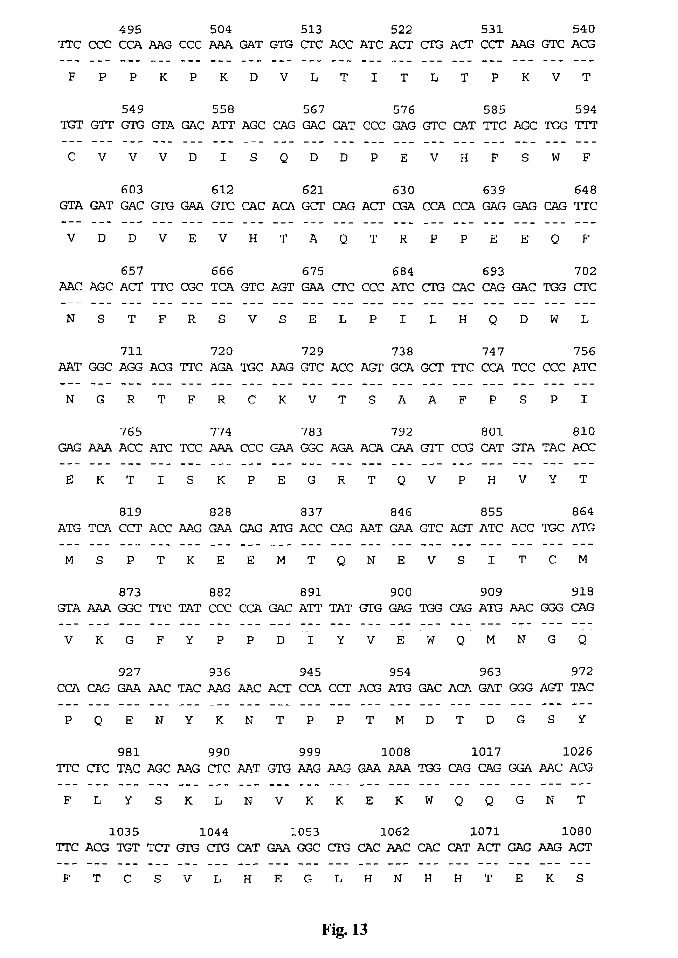

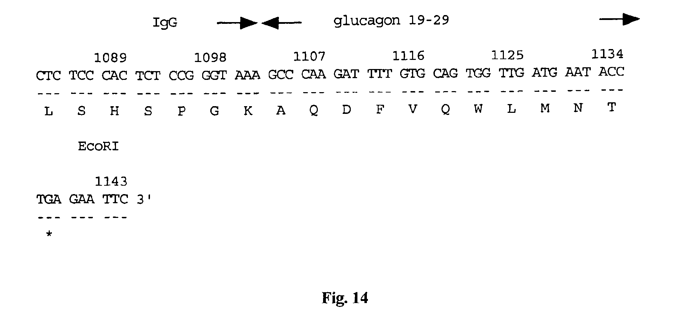

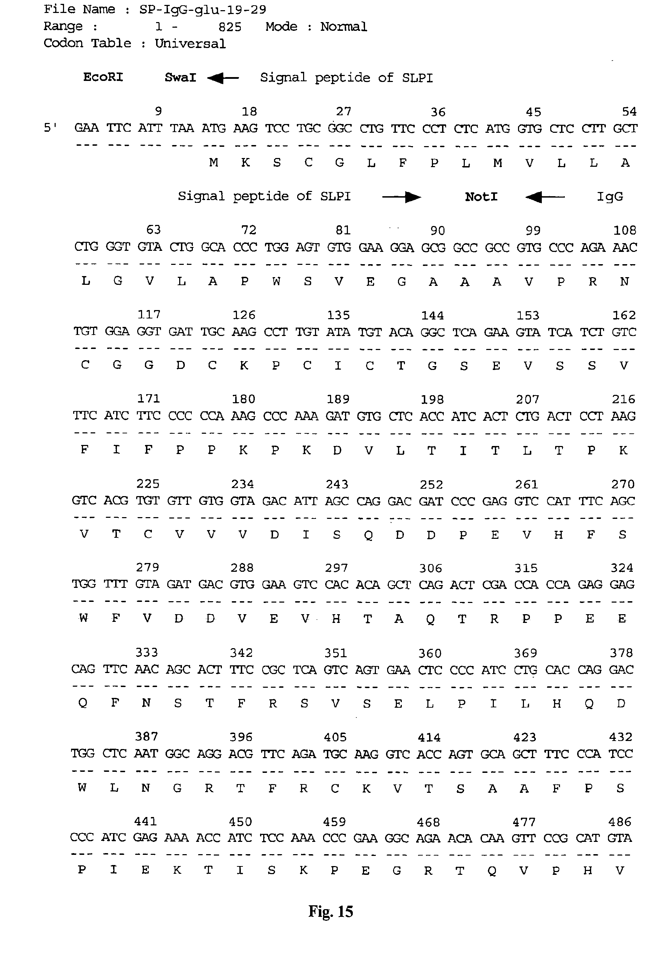

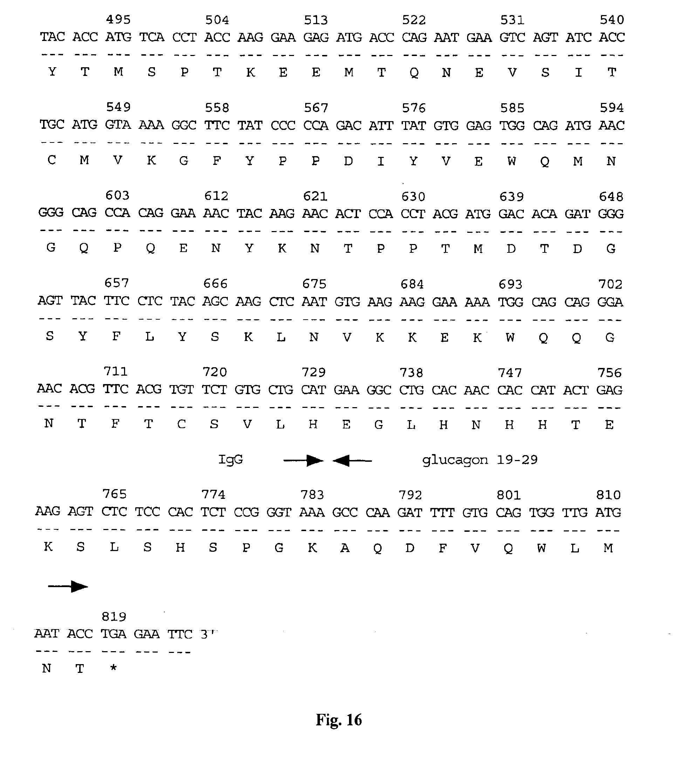

By this, a recombinant vector containing rat IL13-IgG-glucagon¹⁹⁻²⁹(a nucleic acid fragment in which the rat IgG Fc-coding region was ligated to the downstream of rat interleukin 13 (IL13)-coding region, and the glucagon-originated label peptide-coding region was ligated to the downstream of the rat IgG Fc-coding region) having the nucleotide sequence shown in Figs. 12 to 14 and in SEQ ID NO:5 (the restriction sites are also shown), inserted into the EcoRI site of the above-described expression vector pCAGGS for mammalian cells, which recombinant vector expresses IL13-IgG-glucagon ¹⁹⁻²⁹ in rat cells was prepared (Example 3). For comparison, a recombinant vector in which SP-IgG-glucagon ¹⁹⁻²⁹-coding region alone (SEQ ID NO:6 and Figs. 15 and 16) not containing the IL13-coding region was inserted was also prepared (Comparative Example 3).

To rats suffering from autoimmune myocarditis (A novel experimental model of giant cell myocarditis induced in rats by immunization with cardiac myosin fraction, Clinical Immunology and Immunopathology, November 1990, volume 57, p250-262), the recombinant vector was administered in the same manner as in Example 1, and the blood level was measured. On Day 16 from the administration, the animals were sacrificed and anatomized. The percentage of the area of the myocarditis lesion was also checked.

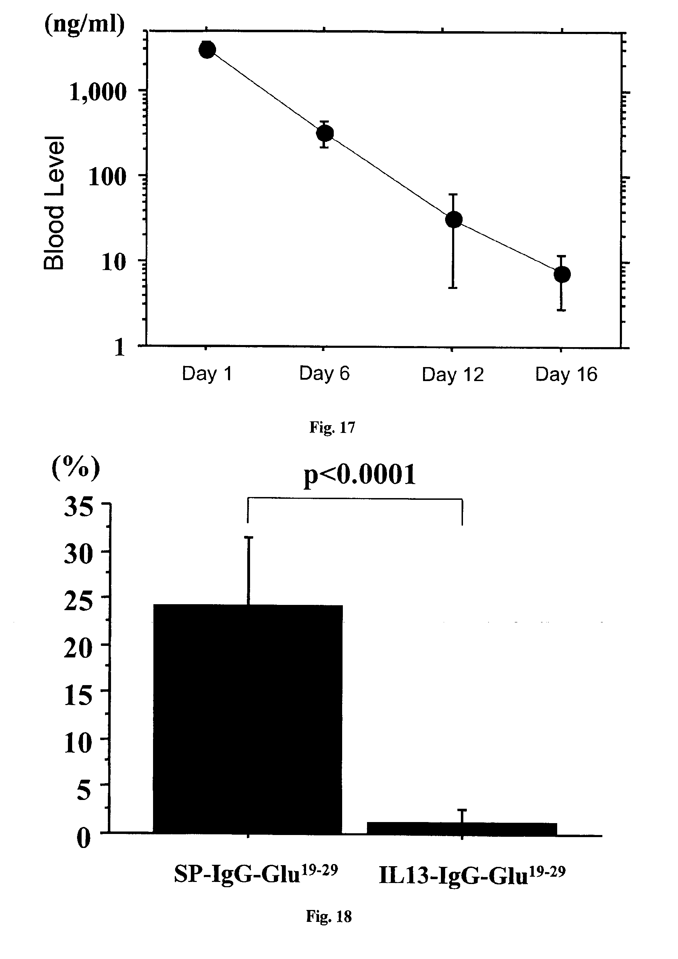

The results are shown in Figs. 17 and 18. The IL13-IgG-glucagon¹⁹⁻²⁹ protein level changed as shown in Fig. 17. That is, on Day 1, a level exceeding 2000 ng/ml was observed. Although the protein level gradually decreased thereafter, the protein level was kept at more than about 8 ng/ml until the rats were sacrificed for evaluation on Day 16. As shown in Fig. 18, in the group to which pCAGGS-IL13-IgG-glucagon¹⁹⁻²⁹ (the vector pCAGGS in which IL13-IgG-glucagon¹⁹⁻²⁹ was inserted) that is a vector for gene therapy according to the present invention, the area of the myocarditis lesion was significantly smaller than in the group to which pCAGGS-SP-IgG-glucagon¹⁹⁻²⁹ not containing IL13 was administered. Thus, the effectiveness of the therapy by pCAGGS-IL13-IgG-glucagon¹⁹⁻²⁹ was shown.

### Example 4, Comparative Example 4

PCR was carried out using as a template the cDNA of cultured mouse spleen cells stimulated by lectin, and using as primers 5'-gagaattcatttaaatggaaatctgctggggaccctac-3' and 5'-gcagcatcgcggccgcttggtcttcctggaagtagaactt-3'. The obtained PCR product was incorporated into pCAGGS-IgG-glu 19-29 prepared by the method described in Example 1, using SwaI and NotI.

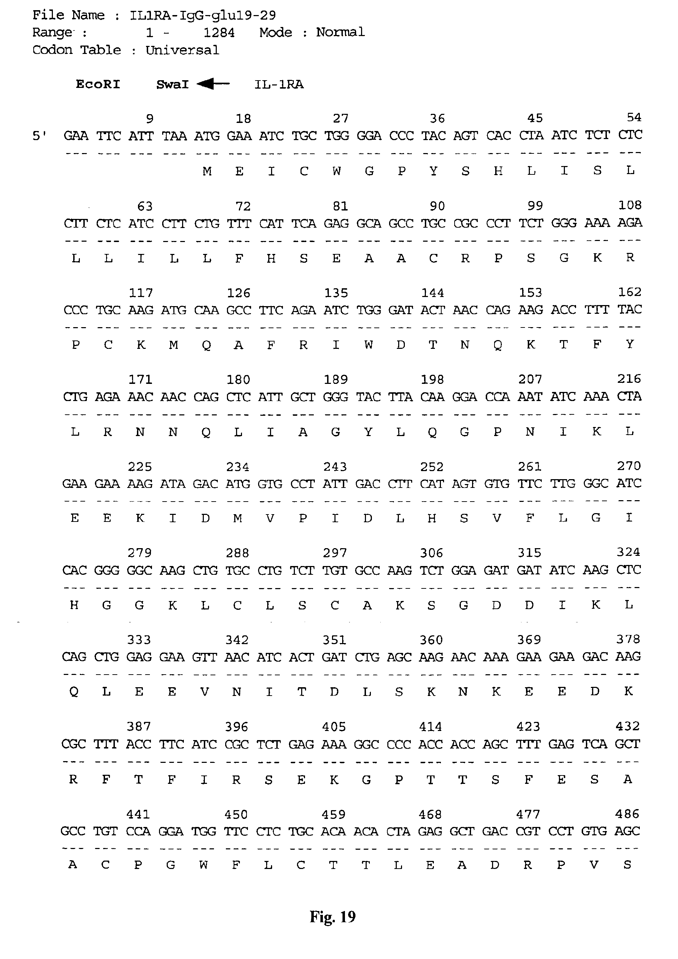

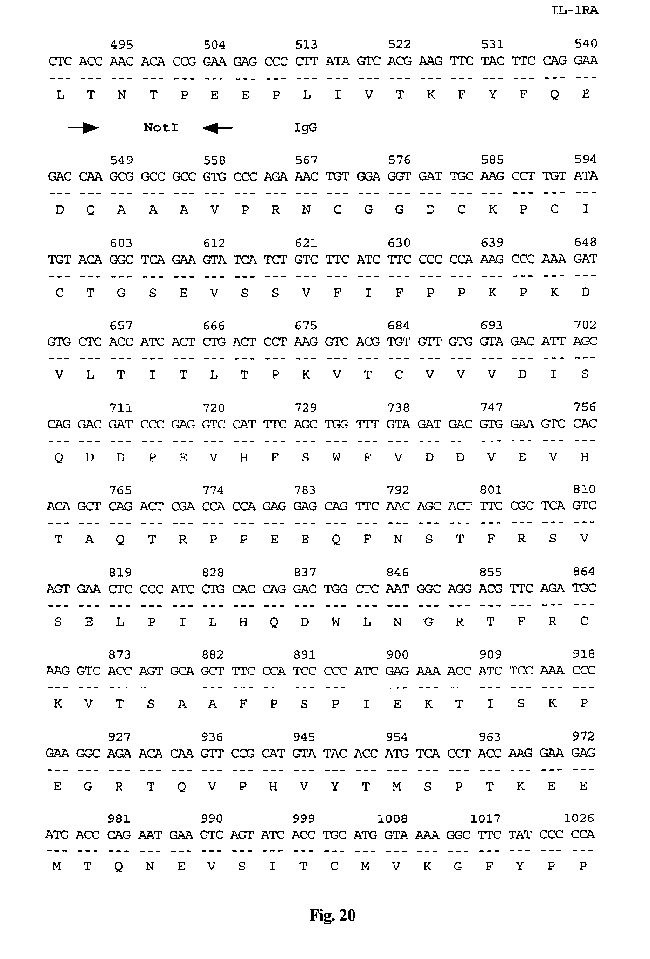

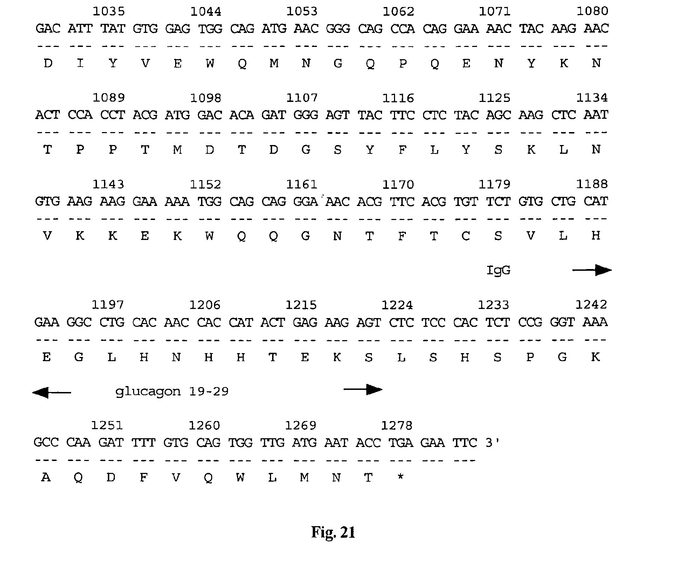

By this, a recombinant vector containing rat IL1RA-IgG-glucagon¹⁹⁻²⁹(a nucleic acid fragment in which the rat IgG Fc-coding region was ligated to the downstream of interleukin 1 receptor antagonist-coding region, and the glucagon-originated label peptide-coding region was ligated to the downstream of the rat IgG Fc-coding region) having the nucleotide sequence shown in Figs. 19 to 21 and in SEQ ID NO:7 (the restriction sites are also shown), inserted into the EcoRI site of the above-described expression vector pCAGGS for mammalian cells, which recombinant vector expresses IL 1RA-IgG-glucagon ¹⁹⁻²⁹ in rat cells was prepared (Example 4).

To rats suffering from autoimmune myocarditis (A novel experimental model of giant cell myocarditis induced in rats by immunization with cardiac myosin fraction, Clinical Immunology and Immunopathology, November 1990, volume 57, p250-262), the recombinant vector was administered in the same manner as in Example 1, and the blood level was measured. On Day 16 from the administration, the animals were sacrificed and anatomized. The percentage of the area of the myocarditis lesion was also checked. For comparison, the above-described vector of Comparative Example 3 was also administered (Comparative Example 4).

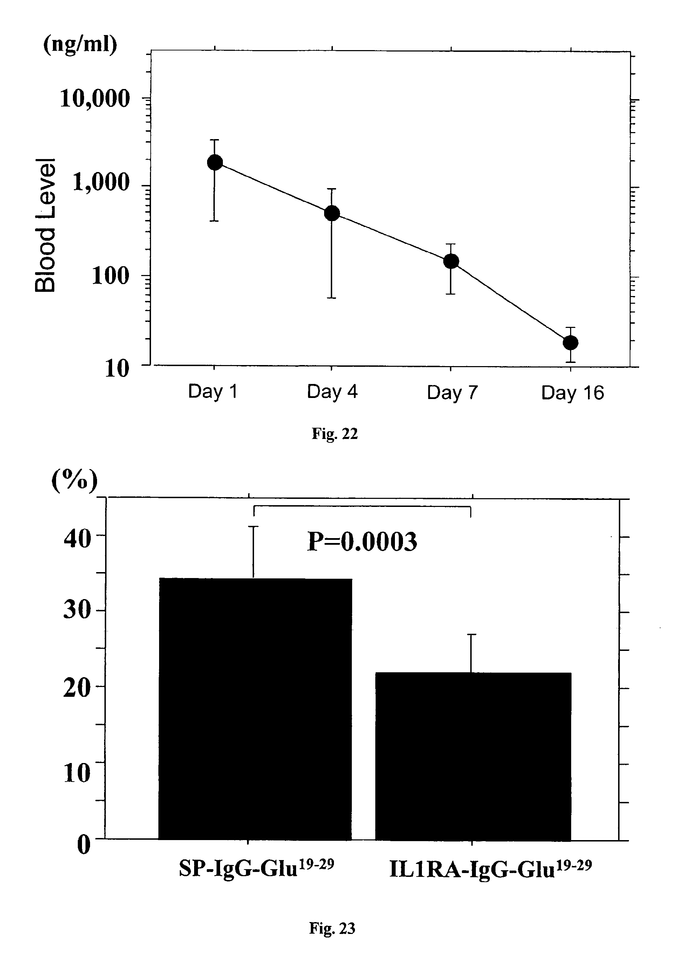

The results are shown in Figs. 22 and 23. The IL1RA-IgG-glucagon¹⁹⁻²⁹ protein level changed as shown in Fig. 22. That is, on Day 1, a level exceeding 2000 ng/ml was observed. Although the protein level gradually decreased thereafter, the protein level was kept at more than about 20 ng/ml until the rats were sacrificed for evaluation on Day 16. As shown in Fig. 23, in Example 4, the area of the myocarditis lesion was significantly smaller than in Comparative Example 4, so that the effectiveness of the therapy by pCAGGS-IL1RA-IgG-glucagon¹⁹⁻²⁹ was shown.

### Example 5

To prepare pCAGGS-glu 19-29 having SwaI and NotI restriction sites, PCR was performed using as primers 5'-gagaattcatttaaatgagagcggccgccccgggtaaagcccaagattttgtgcagtggttg-3' and 5'-gagagagagaattctcaggtattcatcaaccactgcacaaaatcttgggc-3' alone, and the PCR product was inserted into the cloning site of pCAGGS using EcoRI.

Thereafter, PCR was performed using the cDNA of Cos7 cells as a template and using as primers 5'-gagaattcatttaaatgacttccaagctggccgtggct-3' and 5'-gcagcatcgcggccgctgaattctcagccctcttcaaaaa-3'. The PCR product was incorporated into the pCAGGS-glu19-29 prepared above using SwaI and NotI.

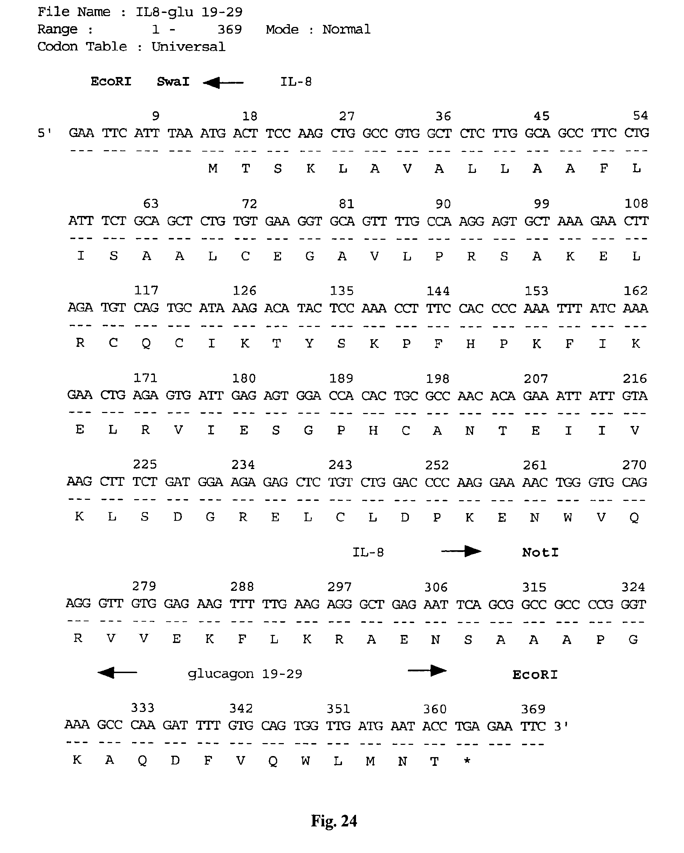

By this, a recombinant vector containing human IL8-glucagon¹⁹⁻²⁹ (a nucleic acid fragment in which the glucagon-originated label peptide-coding region was ligated to the downstream of the human interleukin 8-coding region) having the nucleotide sequence shown in Figs. 24 and in SEQ ID NO:8, inserted into the EcoRI site of the above-described expression vector pCAGGS for mammalian cells, which recombinant vector expresses IL8-glucagon¹⁹⁻²⁹ in rat cells was prepared (Example 5).

The recombinant vector was administered to rats in the same manner as in Example 1. One day after, the blood level was measured. The human IL-8 level in the same sample was also quantified. The quantification of human IL-8 was carried out using IL-8 ELISA kit commercially available from BIOSOURSE (Nivelles, Belgium) in accordance with the Protocols of the product.

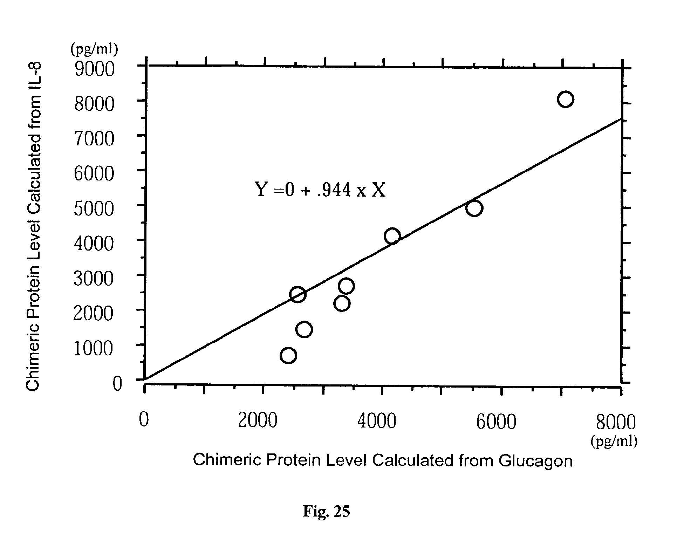

The results are shown in Fig. 25. As shown in Fig. 25, the molar concentrations determined by the both measurements were substantially identical, so that the accuracy of the method using the label peptide of glucagon¹⁹⁻²⁹ was proved.

## Claims

1. A vector for gene therapy comprising an expression vector for mammalian cells and a nucleic acid coding for a fusion protein of glucagon C-terminal side 19-29 amino acid peptide region and a desired protein region which should be produced in the body, which vector can produce said fusion protein in the mammalian cells.

2. The vector according to claim 1, wherein said glucagon C-terminal side 19-29 amino acid peptide region is ligated to the C-terminal of said desired protein region.

3. The vector according to claim 1 or 2, wherein said desired protein is a cytokine, a fusion protein comprising a cytokine and a constant region of immunoglobulin ligated to said cytokine, a growth factor, a hormone or a cell adhesion factor, or a receptor thereof.

4. The vector according to claim 3, wherein said cytokine or the receptor thereof is selected from the group consisting of interferons and receptors thereof, CTLA4, interleukins and receptors thereof.

5. A method for gene therapy comprising administering an effective amount of said vector for gene therapy according to any one of claims 1 to 4 to a mammal or cultured mammalian cells, in which expression of said desired protein in the body or in the cultured mammalian cells is desired.

6. The method according to claim5 , wherein said vector for gene therapy is administered to a mammal.

7. Use of the vector for gene therapy according to any one of claims 1 to 4 for the production of a drug for gene therapy.

8. A method for quantifying a desired protein produced in the body or in cultured cells by expression of said vector for gene therapy, comprising quantifying, by immunoassay, said glucagon C-terminal side 19-29 amino acid peptide region in a test sample collected from a mammal or cultured mammalian cells to which said vector for gene therapy according to any one of claims 1 to 4 was administered.

9. The method according to claim 8, wherein said test sample is collected from said mammal to which said vector for gene therapy was administered.

10. The method according to claim 9, wherein said test sample is a blood sample.

11. A label for labeling a desired protein produced by expression of an externally administered expression vector in the body of a mammal or in cultured mammalian cells, consisting essentially of glucagon C-terminal side 19-29 amino acid peptide.

12. A label for labeling a desired protein produced by expression of an externally administered expression vector in the body of a mammal, consisting essentially of glucagon C-terminal side 19-29 amino acid peptide.

13. A method for labeling a protein produced in the body or in cultured cells, comprising labeling a desired protein produced in the body or in cultured cells with glucagon C-terminal side 19-29 amino acid peptide by expressing said desired protein produced by expression of an externally administered expression vector in the body of a mammal or in cultured mammalian cells, as a fusion protein with said glucagon C-terminal side 19-29 amino acid peptide as a label.

14. The method according to claim 13, comprising labeling said desired protein produced in the body with glucagon C-terminal side 19-29 amino acid peptide by expressing said desired protein produced by expression of an externally administered expression vector in the body of a mammal, as a fusion protein with said glucagon C-terminal side 19-29 amino acid peptide as a label.

15. Use of glucagon C-terminal side 19-29 amino acid peptide as a label for a desired protein produced by expression of an externally administered expression vector in the body of a mammal or in cultured mammalian cells.

16. Use of glucagon C-terminal side 19-29 amino acid peptide as a label for a desired protein produced by expression of an externally administered expression vector in the body of a mammal.


SEQUENCE LISTING

<110>  NIIGATA TLO CORPORATION

<120>  Vector for gene therapy and method for quantifying target protein in
mammal or cultured cell to which the vector for gene therapy was administered

<130>  RXT/FP6316558

<140>  EP 03768321.6
<141>  2003-12-26

<150>  PCT/JP2003/016956
<151>  2003-12-26

<150>  JP 2003-3967
<151>  2003-01-10

<160>  24

<210>  1
<211>  11
<212>  PRT
<213>  Artificial Sequence
<220>
<223>  oligopeptide encoding C19-29 region of glucagon of human, mouse or rat

<400>  1

Ala Gln Asp Phe Val Gln Trp Leu Met Asn Thr
1               5                   10


<210>  2
<211>  1471
<212>  DNA
<213>  Artificial Sequence
<220>
<221>  CDS
<222>  (13)..(1461)
<223>  DNA insert encoding rat IFN-r receptor, rat IgG Fc region and glucagon
C19-29
region

<400>  2
gaattcattt aa atg att ctg ctg gtg gtc ctg atg ctg tct gcg gag atc      51
              Met Ile Leu Leu Val Val Leu Met Leu Ser Ala Glu Ile
              1               5                   10

ggg agt gga gct ttg atg agc acc gag gat cct aag ccg ccc tcg gtg       99
Gly Ser Gly Ala Leu Met Ser Thr Glu Asp Pro Lys Pro Pro Ser Val
    15                  20                  25

cct gcg cca aca aat gtt cta att acg tcc tat gac ttg aac cct gtc      147
Pro Ala Pro Thr Asn Val Leu Ile Thr Ser Tyr Asp Leu Asn Pro Val
30                  35                  40                  45

gta cat tgg aag cac cag aac gtg tcg cag gct gcc gtc ttc act gta      195
Val His Trp Lys His Gln Asn Val Ser Gln Ala Ala Val Phe Thr Val
                50                  55                  60

cag gta aag atg tat cca gaa tac tgg act gat gcc tgc acc aac att 243
Gln Val Lys Met Tyr Pro Glu Tyr Trp Thr Asp Ala Cys Thr Asn Ile
            65                  70                  75

gcc cat cat tat tgt aat atc tac aaa cac att tcc tat cct gac tca 291
Ala His His Tyr Cys Asn Ile Tyr Lys His Ile Ser Tyr Pro Asp Ser
        80                  85                  90

tct gcc tgg gcc aga gtt aag gcc aag gtt gga caa aga gaa tct gcc 339
Ser Ala Trp Ala Arg Val Lys Ala Lys Val Gly Gln Arg Glu Ser Ala
    95                  100                 105

tat gcg cag tca gaa gag ttt att atg tgc cga aag ggg aag gtt gga 387
Tyr Ala Gln Ser Glu Glu Phe Ile Met Cys Arg Lys Gly Lys Val Gly
110                 115                 120                 125

ccg cct ggc ctg gac atc gga agg aag gaa gat cag ctg att gtc cac 435
Pro Pro Gly Leu Asp Ile Gly Arg Lys Glu Asp Gln Leu Ile Val His
                130                 135                 140

ata ttt cac cct aag gtc aat gtg agt cag gaa acc atg ttt ggt gac 483
Ile Phe His Pro Lys Val Asn Val Ser Gln Glu Thr Met Phe Gly Asp
            145                 150                 155

gga aat acc tgt tac aca ttc gac tac act gtg ttt gtg aaa cat tac 531
Gly Asn Thr Cys Tyr Thr Phe Asp Tyr Thr Val Phe Val Lys His Tyr
        160                 165                 170

agg agt ggg gag atc cta cat aca gaa cat agc gtc cta aaa gaa gat 579
Arg Ser Gly Glu Ile Leu His Thr Glu His Ser Val Leu Lys Glu Asp
    175                 180                 185

tgt agc gaa act ctg tgt gag tta aac atc tca gtg tcc acg ctg aat 627
Cys Ser Glu Thr Leu Cys Glu Leu Asn Ile Ser Val Ser Thr Leu Asn
190                 195                 200                 205

tcc aat tac tgt gtt tca gta gtt gga aag tcg tct ttc tgg caa gtt 675
Ser Asn Tyr Cys Val Ser Val Val Gly Lys Ser Ser Phe Trp Gln Val
                210                 215                 220

aat aca gaa aca tca aaa gac gcc tgt atc ccc ttt ctc cat gat gac 723
Asn Thr Glu Thr Ser Lys Asp Ala Cys Ile Pro Phe Leu His Asp Asp
            225                 230                 235

aga gaa gaa gcg gcc gcc gtg ccc aga aac tgt gga ggt gat tgc aag 771
Arg Glu Glu Ala Ala Ala Val Pro Arg Asn Cys Gly Gly Asp Cys Lys
        240                 245                 250

cct tgt ata tgt aca ggc tca gaa gta tca tct gtc ttc atc ttc ccc 819
Pro Cys Ile Cys Thr Gly Ser Glu Val Ser Ser Val Phe Ile Phe Pro
    255                 260                 265

cca aag ccc aaa gat gtg ctc acc atc act ctg act cct aag gtc acg 867
Pro Lys Pro Lys Asp Val Leu Thr Ile Thr Leu Thr Pro Lys Val Thr
270                 275                 280                 285

tgt gtt gtg gta gac att agc cag gac gat ccc gag gtc cat ttc agc 915
Cys Val Val Val Asp Ile Ser Gln Asp Asp Pro Glu Val His Phe Ser
                290                 295                 300

tgg ttt gta gat gac gtg gaa gtc cac aca gct cag act cga cca cca 963

Trp Phe Val Asp Asp Val Glu Val His Thr Ala Gln Thr Arg Pro Pro
            305                 310                 315

gag gag cag ttc aac agc act ttc cgc tca gtc agt gaa ctc ccc atc 1011
Glu Glu Gln Phe Asn Ser Thr Phe Arg Ser Val Ser Glu Leu Pro Ile
        320                 325                 330

ctg cac cag gac tgg ctc aat ggc agg acg ttc aga tgc aag gtc acc 1059
Leu His Gln Asp Trp Leu Asn Gly Arg Thr Phe Arg Cys Lys Val Thr
    335                 340                 345

agt gca gct ttc cca tcc ccc atc gag aaa acc atc tcc aaa ccc gaa 1107
Ser Ala Ala Phe Pro Ser Pro Ile Glu Lys Thr Ile Ser Lys Pro Glu
350                 355                 360                 365

ggc aga aca caa gtt ccg cat gta tac acc atg tca cct acc aag gaa 1155
Gly Arg Thr Gln Val Pro His Val Tyr Thr Met Ser Pro Thr Lys Glu
                370                 375                 380

gag atg acc cag aat gaa gtc agt atc acc tgc atg gta aaa ggc ttc 1203
Glu Met Thr Gln Asn Glu Val Ser Ile Thr Cys Met Val Lys Gly Phe
            385                 390                 395

tat ccc cca gac att tat gtg gag tgg cag atg aac ggg cag cca cag 1251
Tyr Pro Pro Asp Ile Tyr Val Glu Trp Gln Met Asn Gly Gln Pro Gln
        400                 405                 410

gaa aac tac aag aac act cca cct acg atg gac aca gat ggg agt tac 1299
Glu Asn Tyr Lys Asn Thr Pro Pro Thr Met Asp Thr Asp Gly Ser Tyr
    415                 420                 425

ttc ctc tac agc aag ctc aat gtg aag aag gaa aaa tgg cag cag gga 1347
Phe Leu Tyr Ser Lys Leu Asn Val Lys Lys Glu Lys Trp Gln Gln Gly
430                 435                 440                 445

aac acg ttc acg tgt tct gtg ctg cat gaa ggc ctg cac aac cac cat 1395
Asn Thr Phe Thr Cys Ser Val Leu His Glu Gly Leu His Asn His His
                450                 455                 460

act gag aag agt ctc tcc cac tct ccg ggt aaa gcc caa gat ttt gtg 1443
Thr Glu Lys Ser Leu Ser His Ser Pro Gly Lys Ala Gln Asp Phe Val
            465                 470                 475

cag tgg ttg atg aat acc tgagaattct 1471
Gln Trp Leu Met Asn Thr
        480

<210> 3
<211> 4790
<212> DNA
<213> Artificial Sequence
<220>
<223> DNA sequence of artificial expression vector pCAGGS

<400> 3
gtcgacattg attattgact agttattaat agtaatcaat tacggggtca ttagttcata 60

gcccatatat ggagttccgc gttacataac ttacggtaaa tggcccgcct ggctgaccgc 120

ccaacgaccc ccgcccattg acgtcaataa tgacgtatgt tcccatagta acgccaatag 180

ggactttcca ttgacgtcaa tgggtggact atttacggta aactgcccac ttggcagtac 240

atcaagtgta tcatatgcca agtacgcccc ctattgacgt caatgacggt aaatggcccg 300

cctggcatta tgcccagtac atgaccttat gggactttcc tacttggcag tacatctacg 360

tattagtcat cgctattacc atgggtcgag gtgagcccca cgttctgctt cactctcccc 420

atctcccccc cctccccacc cccaattttg tatttattta ttttttaatt attttgtgca 480

gcgatggggg cggggggggg gggggcgcgc gccaggcggg gcggggcggg gcgaggggcg 540

gggcggggcg aggcggagag gtgcggcggc agccaatcag agcggcgcgc tccgaaagtt 600

tccttttatg gcgaggcggc ggcggcggcg gccctataaa aagcgaagcg cgcggcgggc 660

gggagtcgct gcgttgcctt cgccccgtgc cccgctccgc gccgcctcgc gccgcccgcc 720

ccggctctga ctgaccgcgt tactcccaca ggtgagcggg cgggacggcc cttctcctcc 780

gggctgtaat tagcgcttgg tttaatgacg gctcgtttct tttctgtggc tgcgtgaaag 840

ccttaaaggg ctccgggagg gccctttgtg cgggggggag cggctcgggg ggtgcgtgcg 900

tgtgtgtgtg cgtggggagc gccgcgtgcg gcccgcgctg cccggcggct gtgagcgctg 960

cgggcgcggc gcggggcttt gtgcgctccg cgtgtgcgcg aggggagcgc ggccgggggc 1020

ggtgccccgc ggtgcggggg ggctgcgagg ggaacaaagg ctgcgtgcgg ggtgtgtgcg 1080

tgggggggtg agcagggggt gtgggcgcgg cggtcgggct gtaacccccc cctgcacccc 1140

cctccccgag ttgctgagca cggcccggct tcgggtgcgg ggctccgtgc ggggcgtggc 1200

gcggggctcg ccgtgccggg cggggggtgg cggcaggtgg gggtgccggg cggggcgggg 1260

ccgcctcggg ccggggaggg ctcgggggag gggcgcggcg gccccggagc gccggcggct 1320

gtcgaggcgc ggcgagccgc agccattgcc ttttatggta atcgtgcgag agggcgcagg 1380

gacttccttt gtcccaaatc tggcggagcc gaaatctggg aggcgccgcc gcaccccctc 1440

tagcgggcgc gggcgaagcg gtgcggcgcc ggcaggaagg aaatgggcgg ggagggcctt 1500

cgtgcgtcgc cgcgccgccg tccccttctc catctccagc ctcggggctg ccgcaggggg 1560

acggctgcct tcggggggga cggggcaggg cggggttcgg cttctggcgt gtgaccggcg 1620

gctctagagc ctctgctaac catgttcatg ccttcttctt tttcctacag ctcctgggca 1680

acgtgctggt tgttgtgctg tctcatcatt ttggcaaaga attcctcgag gaattcactc 1740

ctcaggtgca ggctgcctat cagaaggtgg tggctggtgt ggccaatgcc ctggctcaca 1800

aataccactg agatcttttt ccctctgcca aaaattatgg ggacatcatg aagccccttg 1860

agcatctgac ttctggctaa taaaggaaat ttattttcat tgcaatagtg tgttggaatt 1920

ttttgtgtct ctcactcgga aggacatatg ggagggcaaa tcatttaaaa catcagaatg 1980

agtatttggt ttagagtttg gcaacatatg ccatatgctg gctgccatga acaaaggtgg 2040

ctataaagag gtcatcagta tatgaaacag ccccctgctg tccattcctt attccataga 2100

aaagccttga cttgaggtta gatttttttt atattttgtt ttgtgttatt tttttcttta 2160

acatccctaa aattttcctt acatgtttta ctagccagat ttttcctcct ctcctgacta 2220

ctcccagtca tagctgtccc tcttctctta tgaagatccc tcgacctgca gcccaagctt 2280

ggcgtaatca tggtcatagc tgtttcctgt gtgaaattgt tatccgctca caattccaca 2340

caacatacga gccggaagca taaagtgtaa agcctggggt gcctaatgag tgagctaact 2400

cacattaatt gcgttgcgct cactgcccgc tttccagtcg ggaaacctgt cgtgccagcg 2460

gatccgcatc tcaattagtc agcaaccata gtcccgcccc taactccgcc catcccgccc 2520

ctaactccgc ccagttccgc ccattctccg ccccatggct gactaatttt ttttatttat 2580

gcagaggccg aggccgcctc ggcctctgag ctattccaga agtagtgagg aggctttttt 2640

ggaggcctag gcttttgcaa aaagctaact tgtttattgc agcttataat ggttacaaat 2700

aaagcaatag catcacaaat ttcacaaata aagcattttt ttcactgcat tctagttgtg 2760

gtttgtccaa actcatcaat gtatcttatc atgtctggat ccgctgcatt aatgaatcgg 2820

ccaacgcgcg gggagaggcg gtttgcgtat tgggcgctct tccgcttcct cgctcactga 2880

ctcgctgcgc tcggtcgttc ggctgcggcg agcggtatca gctcactcaa aggcggtaat 2940

acggttatcc acagaatcag gggataacgc aggaaagaac atgtgagcaa aaggccagca 3000

aaaggccagg aaccgtaaaa aggccgcgtt gctggcgttt ttccataggc tccgcccccc 3060

tgacgagcat cacaaaaatc gacgctcaag tcagaggtgg cgaaacccga caggactata 3120

aagataccag gcgtttcccc ctggaagctc cctcgtgcgc tctcctgttc cgaccctgcc 3180

gcttaccgga tacctgtccg cctttctccc ttcgggaagc gtggcgcttt ctcaatgctc 3240

acgctgtagg tatctcagtt cggtgtaggt cgttcgctcc aagctgggct gtgtgcacga 3300

accccccgtt cagcccgacc gctgcgcctt atccggtaac tatcgtcttg agtccaaccc 3360

ggtaagacac gacttatcgc cactggcagc agccactggt aacaggatta gcagagcgag 3420

gtatgtaggc ggtgctacag agttcttgaa gtggtggcct aactacggct acactagaag 3480

gacagtattt ggtatctgcg ctctgctgaa gccagttacc ttcggaaaaa gagttggtag 3540

ctcttgatcc ggcaaacaaa ccaccgctgg tagcggtggt ttttttgttt gcaagcagca 3600

gattacgcgc agaaaaaaag gatctcaaga agatcctttg atcttttcta cggggtctga 3660

cgctcagtgg aacgaaaact cacgttaagg gattttggtc atgagattat caaaaaggat 3720

cttcacctag atccttttaa attaaaaatg aagttttaaa tcaatctaaa gtatatatga 3780

gtaaacttgg tctgacagtt accaatgctt aatcagtgag gcacctatct cagcgatctg 3840

tctatttcgt tcatccatag ttgcctgact ccccgtcgtg tagataacta cgatacggga 3900

gggcttacca tctggcccca gtgctgcaat gataccgcga gacccacgct caccggctcc 3960

agatttatca gcaataaacc agccagccgg aagggccgag cgcagaagtg gtcctgcaac 4020

tttatccgcc tccatccagt ctattaattg ttgccgggaa gctagagtaa gtagttcgcc 4080

agttaatagt ttgcgcaacg ttgttgccat tgctacaggc atcgtggtgt cacgctcgtc 4140

gtttggtatg gcttcattca gctccggttc ccaacgatca aggcgagtta catgatcccc 4200

catgttgtgc aaaaaagcgg ttagctcctt cggtcctccg atcgttgtca gaagtaagtt 4260

ggccgcagtg ttatcactca tggttatggc agcactgcat aattctctta ctgtcatgcc 4320

atccgtaaga tgcttttctg tgactggtga gtactcaacc aagtcattct gagaatagtg 4380

tatgcggcga ccgagttgct cttgcccggc gtcaatacgg gataataccg cgccacatag 4440

cagaacttta aaagtgctca tcattggaaa acgttcttcg gggcgaaaac tctcaaggat 4500

cttaccgctg ttgagatcca gttcgatgta acccactcgt gcacccaact gatcttcagc 4560

atcttttact ttcaccagcg tttctgggtg agcaaaaaca ggaaggcaaa atgccgcaaa 4620

aaagggaata agggcgacac ggaaatgttg aatactcata ctcttccttt ttcaatatta 4680

ttgaagcatt tatcagggtt attgtctcat gagcggatac atatttgaat gtatttagaa 4740

aaataaacaa ataggggttc cgcgcacatt tccccgaaaa gtgccacctg 4790

<210> 4
<211> 1233
<212> DNA
<213> Artificial Sequence
<220>
<221> CDS
<222> (13)..(1224)
<223> DNA insert encoding rat CTLA4, rat IgG Fc region and glucagon C19-29
region

<400> 4
gaattcattt aa atg gct tgt ctt gga ctc cag agg tac aaa act cac ctg 51
Met Ala Cys Leu Gly Leu Gln Arg Tyr Lys Thr His Leu
1               5                   10

cag ctg cct tct agg act tgg cct ttt gga gtc ctg ctt tct ctt ctc 99
Gln Leu Pro Ser Arg Thr Trp Pro Phe Gly Val Leu Leu Ser Leu Leu
    15                  20                  25

ttc atc cca atc ttc tct gaa gcc ata caa gtg acc caa cct tca gtg 147
Phe Ile Pro Ile Phe Ser Glu Ala Ile Gln Val Thr Gln Pro Ser Val
30                  35                  40                  45

gtg ttg gcc agc agc cac ggt gtc gcc agc ttt cca tgt gaa tat gca 195
Val Leu Ala Ser Ser His Gly Val Ala Ser Phe Pro Cys Glu Tyr Ala
                50                  55                  60

tct tca cac aac act gat gag gtc cgg gtg acg gtg ctg cgg cag aca     243
Ser Ser His Asn Thr Asp Glu Val Arg Val Thr Val Leu Arg Gln Thr
            65                  70                  75

aat gac caa gtg aca gag gtc tgt gcc acg aca ttc aca gtg aag aac     291
Asn Asp Gln Val Thr Glu Val Cys Ala Thr Thr Phe Thr Val Lys Asn
        80                  85                  90

acg ttg ggc ttc cta gat gac ccc ttc tgc agt ggt acc ttt aat gaa     339
Thr Leu Gly Phe Leu Asp Asp Pro Phe Cys Ser Gly Thr Phe Asn Glu
    95                  100                 105

agc aga gtg aac ctc acc atc caa gga ctg agg gct gct gac acc gga     387
Ser Arg Val Asn Leu Thr Ile Gln Gly Leu Arg Ala Ala Asp Thr Gly
110                 115                 120                 125

ctg tac ttc tgc aag gtg gaa ctc atg tac cca ccg cca tac ttt gtg     435
Leu Tyr Phe Cys Lys Val Glu Leu Met Tyr Pro Pro Pro Tyr Phe Val
                130                 135                 140

ggc atg ggc aac ggg acc cag att tat gtc atc gat cca gaa cca tgc     483
Gly Met Gly Asn Gly Thr Gln Ile Tyr Val Ile Asp Pro Glu Pro Cys
            145                 150                 155

cca gat tca gac gcg gcc gcc gtg ccc aga aac tgt gga ggt gat tgc     531
Pro Asp Ser Asp Ala Ala Ala Val Pro Arg Asn Cys Gly Gly Asp Cys
        160                 165                 170

aag cct tgt ata tgt aca ggc tca gaa gta tca tct gtc ttc atc ttc     579
Lys Pro Cys Ile Cys Thr Gly Ser Glu Val Ser Ser Val Phe Ile Phe
    175                 180                 185

ccc cca aag ccc aaa gat gtg ctc acc atc act ctg act cct aag gtc     627
Pro Pro Lys Pro Lys Asp Val Leu Thr Ile Thr Leu Thr Pro Lys Val
190                 195                 200                 205

acg tgt gtt gtg gta gac att agc cag gac gat ccc gag gtc cat ttc     675
Thr Cys Val Val Val Asp Ile Ser Gln Asp Asp Pro Glu Val His Phe
                210                 215                 220

agc tgg ttt gta gat gac gtg gaa gtc cac aca gct cag act cga cca     723
Ser Trp Phe Val Asp Asp Val Glu Val His Thr Ala Gln Thr Arg Pro
            225                 230                 235

cca gag gag cag ttc aac agc act ttc cgc tca gtc agt gaa ctc ccc     771
Pro Glu Glu Gln Phe Asn Ser Thr Phe Arg Ser Val Ser Glu Leu Pro
        240                 245                 250

atc ctg cac cag gac tgg ctc aat ggc agg acg ttc aga tgc aag gtc     819
Ile Leu His Gln Asp Trp Leu Asn Gly Arg Thr Phe Arg Cys Lys Val
    255                 260                 265

acc agt gca gct ttc cca tcc ccc atc gag aaa acc atc tcc aaa ccc     867
Thr Ser Ala Ala Phe Pro Ser Pro Ile Glu Lys Thr Ile Ser Lys Pro
270                 275                 280                 285

gaa ggc aga aca caa gtt ccg cat gta tac acc atg tca cct acc aag     915
Glu Gly Arg Thr Gln Val Pro His Val Tyr Thr Met Ser Pro Thr Lys
                290                 295                 300

gaa gag atg acc cag aat gaa gtc agt atc acc tgc atg gta aaa ggc     963

Glu Glu Met Thr Gln Asn Glu Val Ser Ile Thr Cys Met Val Lys Gly
            305                 310                 315

ttc tat ccc cca gac att tat gtg gag tgg cag atg aac ggg cag cca 1011
Phe Tyr Pro Pro Asp Ile Tyr Val Glu Trp Gln Met Asn Gly Gln Pro
        320                 325                 330

cag gaa aac tac aag aac act cca cct acg atg gac aca gat ggg agt 1059
Gln Glu Asn Tyr Lys Asn Thr Pro Pro Thr Met Asp Thr Asp Gly Ser
    335                 340                 345

tac ttc ctc tac agc aag ctc aat gtg aag aag gaa aaa tgg cag cag 1107
Tyr Phe Leu Tyr Ser Lys Leu Asn Val Lys Lys Glu Lys Trp Gln Gln
350                 355                 360                 365

gga aac acg ttc acg tgt tct gtg ctg cat gaa ggc ctg cac aac cac 1155
Gly Asn Thr Phe Thr Cys Ser Val Leu His Glu Gly Leu His Asn His
                370                 375                 380

cat act gag aag agt ctc tcc cac tct ccg ggt aaa gcc caa gat ttt 1203
His Thr Glu Lys Ser Leu Ser His Ser Pro Gly Lys Ala Gln Asp Phe
            385                 390                 395

gtg cag tgg ttg atg aat acc tgagaattc 1233
Val Gln Trp Leu Met Asn Thr
        400

<210> 5
<211> 1143
<212> DNA
<213> Artificial Sequence

<220>
<221> CDS
<222> (13)..(1134)
<223> DNA insert encoding rat IL13, rat IgG Fc region and glucagon C19-29
region
<400> 5
gaattcattt aa atg gca ctc tgg gtg act gca gtc ctg gct ctc gct tgc 51
Met Ala Leu Trp Val Thr Ala Val Leu Ala Leu Ala Cys
1               5                   10

ctt ggt ggt ctt gcc acc cca ggg cca gtg cgg aga tcc aca tct ccc 99
Leu Gly Gly Leu Ala Thr Pro Gly Pro Val Arg Arg Ser Thr Ser Pro
    15                  20                  25

cct gtg gcc ctc agg gag ctt atc gag gag ctg agc aac atc aca caa 147
Pro Val Ala Leu Arg Glu Leu Ile Glu Glu Leu Ser Asn Ile Thr Gln
30                  35                  40                  45

gac cag aag act tcc ctg tgc aac agc agc atg gta tgg agc gtg gac 195
Asp Gln Lys Thr Ser Leu Cys Asn Ser Ser Met Val Trp Ser Val Asp
                50                  55                  60

ctg aca gct ggc ggg ttc tgt gca gcc ctg gaa tcc ctg acc aac atc 243
Leu Thr Ala Gly Gly Phe Cys Ala Ala Leu Glu Ser Leu Thr Asn Ile
            65                  70                  75

tcc agt tgc aat gcc atc cac agg acc cag agg ata ttg aat ggc ctc 291
Ser Ser Cys Asn Ala Ile His Arg Thr Gln Arg Ile Leu Asn Gly Leu

80                  85                  90

tgt aac caa aag gcc tcg gat gtg gct tcc agc ccc cca gat acc aaa      339
Cys Asn Gln Lys Ala Ser Asp Val Ala Ser Ser Pro Pro Asp Thr Lys
    95                  100                 105

atc gaa gta gcc cag ttt ata tca aaa ctg ctc aat tac tcc aag caa      387
Ile Glu Val Ala Gln Phe Ile Ser Lys Leu Leu Asn Tyr Ser Lys Gln
110                 115                 120                 125

ctt ttc cgc tat ggc cac gcg gcc gcc gtg ccc aga aac tgt gga ggt      435
Leu Phe Arg Tyr Gly His Ala Ala Ala Val Pro Arg Asn Cys Gly Gly
                130                 135                 140

gat tgc aag cct tgt ata tgt aca ggc tca gaa gta tca tct gtc ttc      483
Asp Cys Lys Pro Cys Ile Cys Thr Gly Ser Glu Val Ser Ser Val Phe
            145                 150                 155

atc ttc ccc cca aag ccc aaa gat gtg ctc acc atc act ctg act cct      531
Ile Phe Pro Pro Lys Pro Lys Asp Val Leu Thr Ile Thr Leu Thr Pro
        160                 165                 170

aag gtc acg tgt gtt gtg gta gac att agc cag gac gat ccc gag gtc      579
Lys Val Thr Cys Val Val Val Asp Ile Ser Gln Asp Asp Pro Glu Val
    175                 180                 185

cat ttc agc tgg ttt gta gat gac gtg gaa gtc cac aca gct cag act      627
His Phe Ser Trp Phe Val Asp Asp Val Glu Val His Thr Ala Gln Thr
190                 195                 200                 205

cga cca cca gag gag cag ttc aac agc act ttc cgc tca gtc agt gaa      675
Arg Pro Pro Glu Glu Gln Phe Asn Ser Thr Phe Arg Ser Val Ser Glu
                210                 215                 220

ctc ccc atc ctg cac cag gac tgg ctc aat ggc agg acg ttc aga tgc      723
Leu Pro Ile Leu His Gln Asp Trp Leu Asn Gly Arg Thr Phe Arg Cys
            225                 230                 235

aag gtc acc agt gca gct ttc cca tcc ccc atc gag aaa acc atc tcc      771
Lys Val Thr Ser Ala Ala Phe Pro Ser Pro Ile Glu Lys Thr Ile Ser
        240                 245                 250

aaa ccc gaa ggc aga aca caa gtt ccg cat gta tac acc atg tca cct      819
Lys Pro Glu Gly Arg Thr Gln Val Pro His Val Tyr Thr Met Ser Pro
    255                 260                 265

acc aag gaa gag atg acc cag aat gaa gtc agt atc acc tgc atg gta      867
Thr Lys Glu Glu Met Thr Gln Asn Glu Val Ser Ile Thr Cys Met Val
270                 275                 280                 285

aaa ggc ttc tat ccc cca gac att tat gtg gag tgg cag atg aac ggg      915
Lys Gly Phe Tyr Pro Pro Asp Ile Tyr Val Glu Trp Gln Met Asn Gly
                290                 295                 300

cag cca cag gaa aac tac aag aac act cca cct acg atg gac aca gat      963
Gln Pro Gln Glu Asn Tyr Lys Asn Thr Pro Pro Thr Met Asp Thr Asp
            305                 310                 315

ggg agt tac ttc ctc tac agc aag ctc aat gtg aag aag gaa aaa tgg     1011
Gly Ser Tyr Phe Leu Tyr Ser Lys Leu Asn Val Lys Lys Glu Lys Trp
        320                 325                 330

cag cag gga aac acg ttc acg tgt tct gtg ctg cat gaa ggc ctg cac     1059
Gln Gln Gly Asn Thr Phe Thr Cys Ser Val Leu His Glu Gly Leu His
    335                 340                 345

aac cac cat act gag aag agt ctc tcc cac tct ccg ggt aaa gcc caa     1107
Asn His His Thr Glu Lys Ser Leu Ser His Ser Pro Gly Lys Ala Gln
350                 355                 360                 365

gat ttt gtg cag tgg ttg atg aat acc tgagaattc                       1143
Asp Phe Val Gln Trp Leu Met Asn Thr
                370

<210>  6
<211>  825
<212>  DNA
<213>  Artificial Sequence
<220>
<221>  CDS
<222>  (13)..(816)
<223>  DNA insert encoding rat signal peptide, rat IgG Fc region and glucagon
C19-29
region

<400>  6
gaattcattt aa atg aag tcc tgc ggc ctg ttc cct ctc atg gtg ctc ctt      51
              Met Lys Ser Cys Gly Leu Phe Pro Leu Met Val Leu Leu
              1               5                   10

gct ctg ggt gta ctg gca ccc tgg agt gtg gaa gga gcg gcc gcc gtg      99
Ala Leu Gly Val Leu Ala Pro Trp Ser Val Glu Gly Ala Ala Ala Val
    15                  20                  25

ccc aga aac tgt gga ggt gat tgc aag cct tgt ata tgt aca ggc tca     147
Pro Arg Asn Cys Gly Gly Asp Cys Lys Pro Cys Ile Cys Thr Gly Ser
30                  35                  40                  45

gaa gta tca tct gtc ttc atc ttc ccc cca aag ccc aaa gat gtg ctc     195
Glu Val Ser Ser Val Phe Ile Phe Pro Pro Lys Pro Lys Asp Val Leu
                50                  55                  60

acc atc act ctg act cct aag gtc acg tgt gtt gtg gta gac att agc     243
Thr Ile Thr Leu Thr Pro Lys Val Thr Cys Val Val Val Asp Ile Ser
            65                  70                  75

cag gac gat ccc gag gtc cat ttc agc tgg ttt gta gat gac gtg gaa     291
Gln Asp Asp Pro Glu Val His Phe Ser Trp Phe Val Asp Asp Val Glu
        80                  85                  90

gtc cac aca gct cag act cga cca cca gag gag cag ttc aac agc act     339
Val His Thr Ala Gln Thr Arg Pro Pro Glu Glu Gln Phe Asn Ser Thr
    95                  100                 105

ttc cgc tca gtc agt gaa ctc ccc atc ctg cac cag gac tgg ctc aat     387
Phe Arg Ser Val Ser Glu Leu Pro Ile Leu His Gln Asp Trp Leu Asn
110                 115                 120                 125

ggc agg acg ttc aga tgc aag gtc acc agt gca gct ttc cca tcc ccc     435
Gly Arg Thr Phe Arg Cys Lys Val Thr Ser Ala Ala Phe Pro Ser Pro
                130                 135                 140

atc gag aaa acc atc tcc aaa ccc gaa ggc aga aca caa gtt ccg cat      483
Ile Glu Lys Thr Ile Ser Lys Pro Glu Gly Arg Thr Gln Val Pro His
            145                 150                 155

gta tac acc atg tca cct acc aag gaa gag atg acc cag aat gaa gtc      531
Val Tyr Thr Met Ser Pro Thr Lys Glu Glu Met Thr Gln Asn Glu Val
        160                 165                 170

agt atc acc tgc atg gta aaa ggc ttc tat ccc cca gac att tat gtg      579
Ser Ile Thr Cys Met Val Lys Gly Phe Tyr Pro Pro Asp Ile Tyr Val
    175                 180                 185

gag tgg cag atg aac ggg cag cca cag gaa aac tac aag aac act cca      627
Glu Trp Gln Met Asn Gly Gln Pro Gln Glu Asn Tyr Lys Asn Thr Pro
190                 195                 200                 205

cct acg atg gac aca gat ggg agt tac ttc ctc tac agc aag ctc aat      675
Pro Thr Met Asp Thr Asp Gly Ser Tyr Phe Leu Tyr Ser Lys Leu Asn
                210                 215                 220

gtg aag aag gaa aaa tgg cag cag gga aac acg ttc acg tgt tct gtg      723
Val Lys Lys Glu Lys Trp Gln Gln Gly Asn Thr Phe Thr Cys Ser Val
            225                 230                 235

ctg cat gaa ggc ctg cac aac cac cat act gag aag agt ctc tcc cac      771
Leu His Glu Gly Leu His Asn His His Thr Glu Lys Ser Leu Ser His
        240                 245                 250

tct ccg ggt aaa gcc caa gat ttt gtg cag tgg ttg atg aat acc          816
Ser Pro Gly Lys Ala Gln Asp Phe Val Gln Trp Leu Met Asn Thr
    255                 260                 265

tgagaattc                                                            825


<210>  7
<211>  1284
<212>  DNA
<213>  Artificial Sequence

<220>
<221>  CDS
<222>  (13)..(1275)
<223>  DNA insert encoding rat IL1 receptor antagonist, rat IgG Fc region and
glucagon
C19-29 region

<400>  7
gaattcattt aa atg gaa atc tgc tgg gga ccc tac agt cac cta atc tct      51
              Met Glu Ile Cys Trp Gly Pro Tyr Ser His Leu Ile Ser
              1               5                   10

ctc ctt ctc atc ctt ctg ttt cat tca gag gca gcc tgc cgc cct tct      99
Leu Leu Leu Ile Leu Leu Phe His Ser Glu Ala Ala Cys Arg Pro Ser
    15                  20                  25

ggg aaa aga ccc tgc aag atg caa gcc ttc aga atc tgg gat act aac      147
Gly Lys Arg Pro Cys Lys Met Gln Ala Phe Arg Ile Trp Asp Thr Asn
30                  35                  40                  45

cag aag acc ttt tac ctg aga aac aac cag ctc att gct ggg tac tta      195

Gln Lys Thr Phe Tyr Leu Arg Asn Asn Gln Leu Ile Ala Gly Tyr Leu
                50                  55                  60

caa gga cca aat atc aaa cta gaa gaa aag ata gac atg gtg cct att      243
Gln Gly Pro Asn Ile Lys Leu Glu Glu Lys Ile Asp Met Val Pro Ile
            65                  70                  75

gac ctt cat agt gtg ttc ttg ggc atc cac ggg ggc aag ctg tgc ctg      291
Asp Leu His Ser Val Phe Leu Gly Ile His Gly Gly Lys Leu Cys Leu
        80                  85                  90

tct tgt gcc aag tct gga gat gat atc aag ctc cag ctg gag gaa gtt      339
Ser Cys Ala Lys Ser Gly Asp Asp Ile Lys Leu Gln Leu Glu Glu Val
    95                  100                 105

aac atc act gat ctg agc aag aac aaa gaa gaa gac aag cgc ttt acc      387
Asn Ile Thr Asp Leu Ser Lys Asn Lys Glu Glu Asp Lys Arg Phe Thr
110                 115                 120                 125

ttc atc cgc tct gag aaa ggc ccc acc acc agc ttt gag tca gct gcc      435
Phe Ile Arg Ser Glu Lys Gly Pro Thr Thr Ser Phe Glu Ser Ala Ala
                130                 135                 140

tgt cca gga tgg ttc ctc tgc aca aca cta gag gct gac cgt cct gtg      483
Cys Pro Gly Trp Phe Leu Cys Thr Thr Leu Glu Ala Asp Arg Pro Val
            145                 150                 155

agc ctc acc aac aca ccg gaa gag ccc ctt ata gtc acg aag ttc tac      531
Ser Leu Thr Asn Thr Pro Glu Glu Pro Leu Ile Val Thr Lys Phe Tyr
        160                 165                 170

ttc cag gaa gac caa gcg gcc gcc gtg ccc aga aac tgt gga ggt gat      579
Phe Gln Glu Asp Gln Ala Ala Ala Val Pro Arg Asn Cys Gly Gly Asp
    175                 180                 185

tgc aag cct tgt ata tgt aca ggc tca gaa gta tca tct gtc ttc atc      627
Cys Lys Pro Cys Ile Cys Thr Gly Ser Glu Val Ser Ser Val Phe Ile
190                 195                 200                 205

ttc ccc cca aag ccc aaa gat gtg ctc acc atc act ctg act cct aag      675
Phe Pro Pro Lys Pro Lys Asp Val Leu Thr Ile Thr Leu Thr Pro Lys
                210                 215                 220

gtc acg tgt gtt gtg gta gac att agc cag gac gat ccc gag gtc cat      723
Val Thr Cys Val Val Val Asp Ile Ser Gln Asp Asp Pro Glu Val His
            225                 230                 235

ttc agc tgg ttt gta gat gac gtg gaa gtc cac aca gct cag act cga      771
Phe Ser Trp Phe Val Asp Asp Val Glu Val His Thr Ala Gln Thr Arg
        240                 245                 250

cca cca gag gag cag ttc aac agc act ttc cgc tca gtc agt gaa ctc      819
Pro Pro Glu Glu Gln Phe Asn Ser Thr Phe Arg Ser Val Ser Glu Leu
    255                 260                 265

ccc atc ctg cac cag gac tgg ctc aat ggc agg acg ttc aga tgc aag      867
Pro Ile Leu His Gln Asp Trp Leu Asn Gly Arg Thr Phe Arg Cys Lys
270                 275                 280                 285

gtc acc agt gca gct ttc cca tcc ccc atc gag aaa acc atc tcc aaa      915
Val Thr Ser Ala Ala Phe Pro Ser Pro Ile Glu Lys Thr Ile Ser Lys

290                 295                 300

ccc gaa ggc aga aca caa gtt ccg cat gta tac acc atg tca cct acc      963
Pro Glu Gly Arg Thr Gln Val Pro His Val Tyr Thr Met Ser Pro Thr
            305                 310                 315

aag gaa gag atg acc cag aat gaa gtc agt atc acc tgc atg gta aaa      1011
Lys Glu Glu Met Thr Gln Asn Glu Val Ser Ile Thr Cys Met Val Lys
        320                 325                 330

ggc ttc tat ccc cca gac att tat gtg gag tgg cag atg aac ggg cag      1059
Gly Phe Tyr Pro Pro Asp Ile Tyr Val Glu Trp Gln Met Asn Gly Gln
    335                 340                 345

cca cag gaa aac tac aag aac act cca cct acg atg gac aca gat ggg      1107
Pro Gln Glu Asn Tyr Lys Asn Thr Pro Pro Thr Met Asp Thr Asp Gly
350                 355                 360                 365

agt tac ttc ctc tac agc aag ctc aat gtg aag aag gaa aaa tgg cag      1155
Ser Tyr Phe Leu Tyr Ser Lys Leu Asn Val Lys Lys Glu Lys Trp Gln
                370                 375                 380

cag gga aac acg ttc acg tgt tct gtg ctg cat gaa ggc ctg cac aac      1203
Gln Gly Asn Thr Phe Thr Cys Ser Val Leu His Glu Gly Leu His Asn
            385                 390                 395

cac cat act gag aag agt ctc tcc cac tct ccg ggt aaa gcc caa gat      1251
His His Thr Glu Lys Ser Leu Ser His Ser Pro Gly Lys Ala Gln Asp
        400                 405                 410

ttt gtg cag tgg ttg atg aat acc tgagaattc                            1284
Phe Val Gln Trp Leu Met Asn Thr
    415                 420


<210>  8
<211>  369
<212>  DNA
<213>  Artificial Sequence

<220>
<221>  CDS
<222>  (13)..(360)
<223>  DNA insert encoding human IL8 and glucagon C19-29 region

<400>  8
gaattcattt aa atg act tcc aag ctg gcc gtg gct ctc ttg gca gcc ttc      51
ctg att tct gca gct ctg tgt gaa ggt gca gtt ttg cca agg agt gct      99
aaa gaa ctt aga tgt cag tgc ata aag aca tac tcc aaa cct ttc cac      147
ccc aaa ttt atc aaa gaa ctg aga gtg att gag agt gga cca cac tgc      195
gcc aac aca gaa att att gta aag ctt tct gat gga aga gag ctc tgt      243
ctg gac ccc aag gaa aac tgg gtg cag agg gtt gtg gag aag ttt ttg      291
aag agg gct gag aat tca gcg gcc gcc ccg ggt aaa gcc caa gat ttt      339
gtg cag tgg ttg atg aat acc tgagaattc                                369


<210>  9
<211>  45
<212>  DNA
<213>  Artificial Sequence
<220>
<223>  Oligonucleotide primer used for constructing a vector pCAGGS-IgG-glu19-29

<400>  9
gagaattcat ttaaatgaga gcggccgccg tgcccagaaa ctgtg                    45


<210>  10
<211>  49
<212>  DNA
<213>  Artificial Sequence
<220>
<223>  Oligonucleotide primer used for constructing a vector pCAGGS-IgG-glu19-29

<400>  10
tcaaccactg cacaaaatct tgggctttac ccggagagtg ggagagact                49


<210>  11
<211>  45
<212>  DNA
<213>  Artificial Sequence
<220>
<223>  Oligonucleotide primer used for constructing a vector pCAGGS-IgG-glu19-29

<400>  11
gagaattcat ttaaatgaga gcggccgccg tgcccagaaa ctgtg                    45


<210>  12
<211>  50
<212>  DNA
<213>  Artificial Sequence
<220>
<223>  Oligonucleotide primer used for constructing a vector pCAGGS-IgG-glu19-29

<400>  12
gagagagaga attctcaggt attcatcaac cactgcacaa aatcttgggc               50

<210> 13
<211> 38
<212> DNA
<213> Artificial Sequence
<220>
<223> Oligonucleotide primer used for constructing a vector
pCAGGS-IFN-rR-IgG-glu19-29

<400> 13
gagaattcat ttaaatgatt ctgctggtgg tcctgatg 38


<210> 14
<211> 40
<212> DNA
<213> Artificial Sequence
<220>
<223> Oligonucleotide primer used for constructing a vector
pCAGGS-IFN-rR-IgG-glu19-29

<400> 14
gcagcatcgc ggccgcttct tctctgtcat catggagaaa 40


<210> 15
<211> 38
<212> DNA
<213> Artificial Sequence
<220>
<223> Oligonucleotide primer used for constructing a vector
pCAGGS-CTLA4-IgG-glu19-29

<400> 15
gagaattcat ttaaatggct tgtcttggac tccagagg 38


<210> 16
<211> 40
<212> DNA
<213> Artificial Sequence
<220>
<223> Oligonucleotide primer used for constructing a vector
pCAGGS-CTLA4-IgG-glu19-29

<400> 16
gcagcatcgc ggccgcgtct gaatctgggc atggttctgg 40


<210> 17
<211> 38
<212> DNA
<213> Artificial Sequence
<220>
<223> Oligonucleotide primer used for constructing a vector
pCAGGS-IL13-IgG-glu19-29

<400> 17
gagaattcat ttaaatggca ctctgggtga ctgcagtc 38

<210> 18
<211> 40
<212> DNA
<213> Artificial Sequence
<220>
<223> Oligonucleotide primer used for constructing a vector
pCAGGS-IL13-IgG-glu19-29

<400> 18
gcagcatcgc ggccgcgtgg ccatagcgga aaagttgctt 40

<210> 19
<211> 38
<212> DNA
<213> Artificial Sequence
<220>
<223> Oligonucleotide primer used for constructing a vector
pCAGGS-IL1RA-IgG-glu19-29

<400> 19
gagaattcat ttaaatggaa atctgctggg gaccctac 38

<210> 20
<211> 40
<212> DNA
<213> Artificial Sequence
<220>
<223> Oligonucleotide primer used for constructing a vector
pCAGGS-IL1RA-IgG-glu19-29

<400> 20
gcagcatcgc ggccgcttgg tcttcctgga agtagaactt 40

<210> 21
<211> 62
<212> DNA
<213> Artificial Sequence
<220>
<223> Oligonucleotide primer used for constructing a vector pCAGGS-glu19-29

<400> 21
gagaattcat ttaaatgaga gcggccgccc cgggtaaagc ccaagatttt gtgcagtggt 60
tg 62

<210> 22
<211> 50
<212> DNA
<213> Artificial Sequence
<220>
<223> Oligonucleotide primer used for constructing a vector pCAGGS-glu19-29

<400> 22
gagagagaga attctcaggt attcatcaac cactgcacaa aatcttgggc 50

<210> 23
<211> 38

<212>  DNA
<213>  Artificial Sequence
<220>
<223>  Oligonucleotide primer used for constructing a vector pCAGGS-IL8-glu19-29

<400>  23
gagaattcat ttaaatgact tccaagctgg ccgtggct                              38


<210>  24
<211>  40
<212>  DNA
<213>  Artificial Sequence
<220>
<223>  Oligonucleotide primer used for constructing a vector pCAGGS-IL8-glu19-29

<400>  24
gcagcatcgc ggccgctgaa ttctcagccc tcttcaaaaa                            40


1/28